(19) Europäisches Patentamt / European Patent Office / Office européen des brevets

(11) EP 2 233 503 A1

(12) **EUROPEAN PATENT APPLICATION**
published in accordance with Art. 153(4) EPC

(43) Date of publication:
**29.09.2010 Bulletin 2010/39**

(21) Application number: **08859171.4**

(22) Date of filing: **15.12.2008**

(51) Int Cl.:
***C07K 16/44*** *(2006.01)* ***C07K 14/76*** *(2006.01)*
***C07K 14/79*** *(2006.01)* ***C12N 5/10*** *(2006.01)*
***C12N 15/02*** *(2006.01)* ***G01N 33/50*** *(2006.01)*
***G01N 33/53*** *(2006.01)* ***G01N 33/577*** *(2006.01)*
***C12P 21/08*** *(2006.01)*

(86) International application number:
**PCT/JP2008/072783**

(87) International publication number:
**WO 2009/075376 (18.06.2009 Gazette 2009/25)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **13.12.2007 JP 2007321529**

(83) **Declaration under Rule 32(1) EPC (expert solution)**

(71) Applicants:
- **Sekisui Medical Co., Ltd.**
  **Tokyo 103-0027 (JP)**
- **Daiichi Sankyo Company, Limited**
  **Chuo-ku**
  **Tokyo 103-8642 (JP)**

(72) Inventors:
- **MIYAZAKI, Osamu**
  **Ryugasaki-shi**
  **Ibaraki 301-0852 (JP)**
- **TAKUBO, Kohei**
  **Ryugasaki-shi**
  **Ibaraki 301-0852 (JP)**
- **FUKAMACHI, Isamu**
  **Ryugasaki-shi**
  **Ibaraki 301-0852 (JP)**
- **KUSAKA, Eriko**
  **Ryugasaki-shi**
  **Ibaraki 301-0852 (JP)**
- **KASHIWAGI, Mieko**
  **Ryugasaki-shi**
  **Ibaraki 301-0852 (JP)**

(74) Representative: **HOFFMANN EITLE**
**Patent- und Rechtsanwälte**
**Arabellastrasse 4**
**81925 München (DE)**

(54) **ANTI-OFLOXACIN MONOCLONAL ANTIBODY AND IMMUNOASSAY METHOD OF OFLOXACIN USING THE SAME**

(57) A method that accurately and conveniently detects a compound shown by the formula (I) in a human sample is disclosed. An antibody that reacts with the compound shown by the formula (I), but does not cross-react with the N-oxide metabolite and the desmethyl levofloxacin metabolite of the compound shown by the formula (I) is prepared using an antigen produced by binding bovine serum albumin to the compound shown by the formula (I) through the 6-position carboxyl group.

Immunoassay that measures the compound shown by the formula (I) that has not been metabolized is implemented using the above antibody.

EP 2 233 503 A1

[Chem 1]

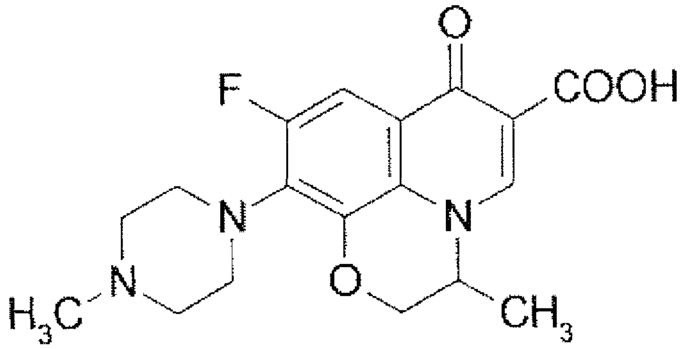

(I)

## Description

TECHNICAL FIELD

The present invention relates to an antibody that reacts with ofloxacin (i.e., antibiotic compound) and each optical isomer that forms ofloxacin, but does not react with the main metabolites thereof.

BACKGROUND ART

New quinolone antibiotics are drugs that exhibit a remarkably improved antibiotic spectrum and antibiotic activity as compared with pyridonecarboxylic acid antibiotics (e.g., enoxacin and norfloxacin), and exhibit high selective toxicity by inhibiting bacterial DNA gyrase. Ofloxacin, levofloxacin, norfloxacin, ciprofloxacin, etc. have been clinically used to treat various infections, purulent diseases, etc., and achieved an excellent therapeutic effect.

Ofloxacin is shown by the following chemical formula (I), and has one asymmetric carbon atom in the chemical structure. Ofloxacin is a racemic body that includes two optically active substances (i.e., S-(-)-isomer and R-(+)-isomer) in a composition ratio of 1:1.

[Chem 1]

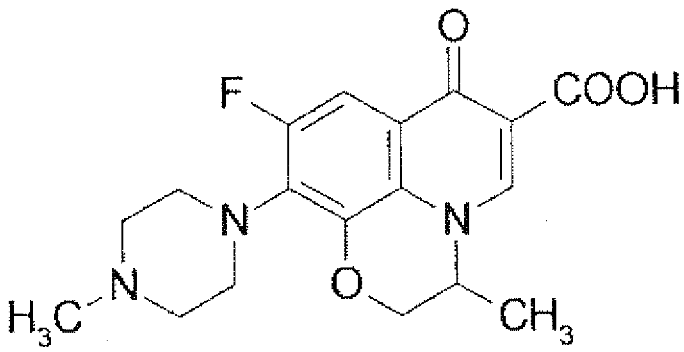

(I)

The racemic body exhibits antibiotic activity mainly due to levofloxacin that is the S-(-)-isomer. The antibiotic activity of levofloxacin is approximately twice that of ofloxacin. It has been known that levofloxacin is highly effective for various infections such as respiratory tract infections and urinary tract infections.

It is important that an appropriate amount of active ingredient is present inside the body so that the antibiotic sufficiently exhibits the antibiotic effect. Therefore, a measurement system that quantitatively determines only a compound that exhibits antibiotic activity present inside the body without measuring compounds (e.g., metabolites) of which the antibiotic activity has weakened or been lost (hereinafter may be referred to as "compounds that have lost the antibiotic activity"), is indispensable for best effectively prescribing antibiotics. As a method that measures only a compound that exhibits antibiotic activity, immunoassay that uses an antibody that specifically recognizes the target antibiotic, a method that separates and analyzes the target antibiotic by HPLC based on the molecular weight and the polarity of the target antibiotic, a method that directly measures the antibiotic activity via culture with bacteria, and the like have been widely used. Among these, immunoassay is advantageous because immunoassay does not require expensive equipment, enables short-time measurement, has excellent sensitivity, and can measure a number of samples.

It has been known that many drugs reversibly bind to biological components such as serum proteins (e.g., albumin), glycoproteins, and lipoproteins (hereinafter may be collectively referred to as "serum proteins") when administered via various routes. Specifically, the concentration of a drug in blood corresponds to the sum of the concentration of the bound drug that is binding to serum proteins and the concentration of the free (unbound) drug that is not binding to serum proteins. In order to accurately measure the concentration of a drug in blood by immunoassay that uses an antibody, it is necessary for the antibody to equally react with the bound drug and the free drug. However, the antibody may not bind to the epitope of the drug due to serum proteins that are bound to the drug so that the concentration of the drug in blood may not be accurately measured.

As a method that detects new quinolone antibiotics by immunoassay, an immunoassay that uses a monoclonal antibody that recognizes a bicyclic new quinolone antibiotic, but does not recognize a tricyclic new quinolone such as

ofloxacin has been disclosed (Patent Document 1). However, the invention disclosed in Patent Document 1 aims at detecting the residual amount of bicyclic new quinolone used for preventing infections of livestock or cultured fish/shellfish, and cannot detect ofloxacin. Moreover, the invention disclosed in Patent Document 1 aims at obtaining an antibody that can simultaneously detect a plurality of types of new quinolone antibiotics (i.e., an antibody that cross-reacts with a plurality of types of new quinolone antibiotics).

Patent Document 2 discloses an antibody of levofloxacin that is the S-isomer of the compound shown by the formula (I), and an immunoassay. The antibody disclosed in Patent Document 2 is a polyclonal antibody that cross-reacts with metabolites (levofloxacin-N-oxide or desmethyl levofloxacin) that have lost the antibiotic activity. Therefore, it is impossible to accurately detect only levofloxacin that maintains the antibiotic activity.

Patent Document 1: JP-A-2007-63180
Patent Document 2: JP-A-7-267999

DISCLOSURE OF THE INVENTION

PROBLEMS TO BE SOLVED BY THE INVENTION

The new quinolone antibiotic compound (e.g., ofloxacin) exhibits very low antigenicity. Therefore, it is difficult to efficiently produce an antibody that recognizes a new quinolone antibiotic compound by directly utilizing the new quinolone antibiotic compound for immunization as an antigen (antigen for immunization) to produce an antibody. Therefore, for producing the antibody that recognizes the new quinolone antibiotic compound, it is appropriate to use an antigen produced by binding, as a carrier, a protein (carrier protein) to the new quinolone antibiotic compound. It has been considered that it is necessary to bind a new quinolone antibiotic compound to a protein without impairing a functional group that affects antigenicity (e.g., the 4-position ketone (carbonyl) group or the 3-position carboxyl group in the quinolone skeleton, or the fluorine atom). Therefore, Patent Document 2 binds a compound obtained by converting the 4-methylpiperazinyl group (10-position substituent) of levofloxacin into a 4-carboxymethylpiperazinyl group to bovine serum albumin (BSA) (i.e., carrier), and uses the resulting product as an antigen for immunization.

However, the antibody obtained by the method disclosed in Patent Document 2 cross-reacts with metabolites (N-oxide or desmethyl metabolite) of levofloxacin. An antibody that reacts only with levofloxacin has never been obtained.

An object of the present invention is to provide an antibody that recognizes the compound shown by the formula (I), but does not recognize (cross-react with) an N-oxide and/or desmethyl metabolite thereof, and a method of producing the same. Another object of the present invention is to provide an immunoassay (e.g., radioimmunoassay, enzyme immunoassay, support (particle) agglutination inhibition immunoassay, and immunochromatography) using the above antibody. A further object of the present invention is to provide an antigen for immunization that is useful for producing the above antibody.

MEANS FOR SOLVING THE PROBLEMS

The inventors of the present invention successfully produced an excellent antigen for immunization that can achieve the above object by binding a carrier protein (e.g., bovine serum albumin) to the carboxyl group (i.e., 6-position substituent) of levofloxacin ((-)-(S)-9-fluoro-2,3-dihydro-3-methyl-10-(4-methyl-1-piperazinyl)-7-oxo-7H-pyrido[1,2,3-de][1,4]benzoxazine-6-carboxylic acid).
The inventors found that a useful antibody that reacts with the compound shown by the formula (I), but does not recognize an N-oxide metabolite and a desmethyl metabolite thereof, can be provided by utilizing the above antigen for immunization. The inventors also found that a useful antibody that recognizes levofloxacin that is the S-isomer of the compound shown by the formula (I), but does not recognize metabolites thereof (i.e., levofloxacin-N-oxide and desmethyl levofloxacin), can be provided by utilizing the above antigen for immunization. The inventors found that a useful antibody that recognizes the R-isomer of the compound shown by the formula (I), but does not recognize an N-oxide metabolite and a desmethyl metabolite thereof, can be provided by utilizing the above antigen for immunization. The inventors completed an immunoassay that measures the compound shown by the formula (I) that has not been metabolized/decomposed using the above antibody. Above findings have led to the completion of the present invention.

Thus, the present invention provides an antibody that reacts with the compound shown by the formula (I), but does not recognize an N-oxide metabolite and a desmethyl metabolite thereof. One aspect of the present invention provides an antigen for immunization that is obtained by binding a carrier protein to levofloxacin that is the S-isomer of the compound shown by the formula (I). Another aspect of the present invention provides an antigen for immunization that is obtained by binding a carrier protein to the carboxyl group (i.e., 6-position substituent) of levofloxacin. Another aspect of the present invention provides a method of producing an antibody that recognizes the S-isomer and the R-isomer of the compound shown by the formula (I), or recognizes only levofloxacin that is the S-isomer of the compound

shown by the formula (I), or recognizes only the R-isomer of the compound shown by the formula (I), but does not recognize an N-oxide metabolite and a desmethyl metabolite thereof, by immunizing an animal using the above antigen, and the antibody produced by the above method. A further aspect of the present invention provides a method of measuring the concentration of the compound shown by the formula (I) in a sample by immunoassay that utilizes said above antibodies.

Specifically, the present invention provides the following.

(1) An antibody that reacts with a compound shown by the following formula (I),

[Chem 2]

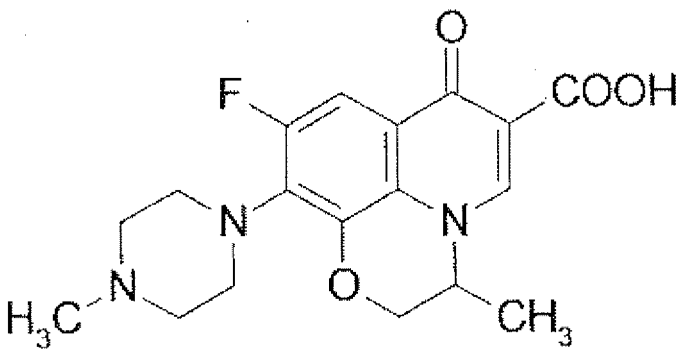

(I)

but does not cross-react with an N-oxide metabolite and/or a desmethyl metabolite thereof.
(2) The antibody according to (1), the antibody strongly reacting with the S-isomer of the compound shown by the formula (I).
(3) The antibody according to (1), the antibody strongly reacting with the R-isomer of the compound shown by the formula (I).
(4) The antibody according to (1), the antibody strongly reacting with racemic body of the compound shown by the formula (I).
(5) The antibody according to (1), the antibody not cross-reacting with one or more compounds selected from the group consisting of diclofenac sodium, nabumetone, flurbiprofen, ketoprofen, loxoprofen sodium, oxaprozin, naproxen, ibuprofen, carbocisteine, salicylamide, acetaminophen, anhydrous caffeine, methylenedisalicylic acid, promethazine, and theophylline.
(6) The antibody according to (1), the antibody not cross-reacting with, or weakly reacting with, new quinolone antibiotics other than the compound shown by the formula (I).
(7) The antibody according to any one of (1) to (6), wherein reactivity of the antibody with the compound shown by the formula (I) does not change due to the presence of a biological sample-derived component.
(8) The antibody according to any one of (1) to (7), wherein the biological sample-derived component is a serum component, a plasma component, or a salivary component.
(9) The antibody according to any one of (1) to (6), wherein reactivity of the antibody with the compound shown by the formula (I) does not change due to binding of the compound shown by the formula (I) and a serum component.
(10) The antibody according to any one of (1) to (9), the antibody being a monoclonal antibody.
(11) The monoclonal antibody according to (10), the monoclonal antibody being specific to the compound shown by the formula (I), and satisfying a reaction condition whereby, in a reaction system in which the compound shown by the formula (I) in a sample and an N-oxide metabolite and/or a desmethyl metabolite of the compound shown by the formula (I) in a sample are present so that an immune reaction between the compound shown by the formula (I) immobilized on a solid phase and the antibody is inhibited, the 50% inhibitory concentration of the compound shown by the formula (I) in the sample is higher than the 50% inhibitory concentration of the N-oxide metabolite and/or the desmethyl metabolite of the compound shown by the formula (I) in the sample.
(12) A hybridoma that produces the monoclonal antibody according to (10) or (11).
(13) An antigen for producing the antibody according to (1), the antigen being produced by binding a carrier protein to the compound shown by the formula (I) through the 6-position carboxyl group.
(14) The antigen according to (13), wherein the carrier protein is BSA or transferrin.

(15) The antigen according to (13) or (14), wherein 12 to 14 molecules of the compound shown by the formula (I) are bound to one molecule of the carrier protein.
(16) An immunization method comprising immunizing an animal with the antigen according to any one of (13) to (15).
(17) An antibody screening method to select a desired antibody comprising: reacting an antibody with the compound shown by the formula (I) in the presence of a compound for which it is desired to determine cross-reactivity, wherein the antibody is obtained by immunizing an animal with the antigen according to any one of (13) to (15), wherein the former compound is immobilized on a solid phase, and; comparing the resulting reactivity with the reactivity in the absence of the latter compound for which it is desired to determine cross-reactivity.
(18) An antibody screening method to select a desired antibody comprising reacting an antibody obtained by immunizing an animal with the antigen according to any one of (13) to (15) with the compound shown by the formula (I) that is immobilized on a solid phase in the presence of a biological sample-derived component, and comparing the resulting reactivity with the reactivity in the absence of the biological sample-derived component.
(19) An immunoassay for detecting the compound shown by the formula (I) in a sample, comprising using the antibody according to any one of (1) to (11).
(20) An immunoassay for detecting the compound shown by the formula (I) in a sample, comprising competitively reacting a synthetic polyvalent antigen and the compound shown by the formula (I) in the sample with the antibody according to any one of (1) to (11) that is immobilized on a solid phase.
(21) An immunoassay for detecting the compound shown by the formula (I) in a sample, comprising competitively reacting an immobilized synthetic polyvalent antigen and the compound shown by the formula (I) in the sample with the antibody according to any one of (1) to (11).
(22) An immunoassay for detecting the compound shown by the formula (I) in a sample, comprising competitively reacting an immobilized synthetic polyvalent antigen and the compound shown by the formula (I) in the sample with the antibody according to any one of (1) to (11) that is immobilized on a solid phase.
(23) The immunoassay according to any one of (19) to (22), wherein the solid phase is a latex particle.
(24) The immunoassay according to any one of (19) to (23), wherein the competitive reaction is measured by an agglutination inhibition method.
(25) The immunoassay according to any one of (19) to (24), wherein the sample is blood, serum, plasma, urine, saliva, sputum, lacrimal fluid, otorrhea, or prostatic fluid.
(26) The immunoassay according to (25), wherein the sample is a sample collected from a patient who has been administered the compound shown by the formula (I).
(27) A reagent for immunoassay for detecting the compound shown by the formula (1) in a sample, the reagent comprising:

(a) the antibody according to any one of (1) to (11), or the antibody according to any one of(1) to (11) that is immobilized on a solid phase; and
(b) a synthetic polyvalent antigen or an immobilized synthetic polyvalent antigen.

EFFECTS OF THE INVENTION

The antibody provided by the present invention recognizes the compound shown by the formula (I) and is useful. The concentration of the compound shown by the formula (I) in various samples (e.g., biological sample) can be measured with high sensitivity by immunoassay that utilizes the antibody.

BEST MODE FOR CARRYING OUT THE INVENTION

The antibody according to the present invention reacts with the compound shown by the formula (I), but does not cross-react with N-oxide- and/or desmethyl-metabolites thereof. Since the antibody according to the present invention reacts with the compound shown by the formula (I), but does not react with metabolites thereof, only the compound shown by the formula (I) that is effectively present in blood as antibiotic can be specifically measured by utilizing the antibody according to the present invention for immunoassay.
The antibody according to the present invention may be an antibody that strongly reacts with levofloxacin that is the S-isomer of the compound shown by the formula (I), an antibody that strongly reacts with the R-isomer of the compound shown by the formula (I), or an antibody that reacts with the R-isomer and the S-isomer of the compound shown by the formula (I).
The antibody according to the present invention may be an antibody that does not react or weakly reacts with concomitant drugs for the compound shown by the formula (I) or analogues of the compound shown by the formula (I).
The antibody according to the present invention may be a polyclonal antibody obtained from serum (antiserum) of an immunized animal, or may be a monoclonal antibody produced from a hybridoma prepared using antibody-producing

cells of an immunized animal.
The meanings of the terms "cross-react", "strongly react", "does not react", and "weakly react" are described later.

An antigen (antigen for immunization) that is used to produce an antibody that recognizes the S-isomer and/or the R-isomer of the compound shown by the formula (I) according to the present invention may be any antigen that produces an antibody that recognizes the compound shown by the formula (I), but does not recognize N-oxide metabolite and desmethyl metabolite thereof. It is suitable to use an antigen that is produced by binding a carrier (e.g., protein) to the carboxyl group (i.e., 6-position substituent) of the compound shown by the formula (I).

A protein that is used as the carrier for the antigen for immunization ("carrier protein") may be appropriately selected from various proteins that are generally considered to be useful for producing antibody to a low-molecular-weight antigen (hapten). The carrier protein and the antigen may be bound by known methods. For example, bovine serum albumin or transferrin may be used as the carrier protein. The carrier protein and the antigen may be bound by utilizing a condensation reaction using dicyclohexylcarbodiimide, or an active ester method. Note that the type of protein used as the carrier and the method of binding the carrier protein and the antigen are not limited thereto.

The number of molecules (binding number) of the compound shown by the formula (I) that are bound to one molecule of the carrier protein is not particularly limited insofar as the resulting antigen is recognized as antigen in the animal to be immunized. For example, an antigen in which 12 to 14 molecules of the compound shown by the formula (I) are bound to one molecule of the carrier protein is desirably used taking account of the antibody production efficiency. Note that the binding number is not limited thereto. The details of the method of preparing antigen for immunization are described later. A desired number of molecules of the compound shown by the formula (I) can be bound by increasing or decreasing the amount of the compound shown by the formula (I) used as a reaction raw material with respect to the amount of the carrier protein. Specifically, the binding number increases when increasing the amount of the compound added as the raw material, and decreases when decreasing the amount of the compound added as the raw material. Note that the term "binding ratio" or "binding content" may be used herein in a sense similar to the term "binding number".

The antigen for immunization according to the present invention produced by the above method may also be used as antigen for screening hybridoma or antibody, or antigen (antigen for competitive reaction) for immunoassay described later. When using an antigen for immunization according to the present invention as antigen for screening or antigen for immunoassay, the antigen may be immobilized on a solid (solid phase) such as an insoluble support, or may be used as a labeled antigen that is labeled with a well-known marker (described later). Such an immobilized (solid-phase) antigen and a labeled antigen are also included within the scope of the present invention. For example, an immobilized (solid-phase) antigen may be produced by causing the antigen to be physically adsorbed on or chemically bound to an insoluble support. The antigen may be chemically bound to the insoluble support through an appropriate spacer.

The insoluble support may be formed of a polymer material (e.g., polystyrene resin), an inorganic material (e.g., glass), a polysaccharide material (e.g., cellulose or agarose), or the like. The shape of the insoluble support is not particularly limited. The insoluble support may be in the shape of plate (e.g., microplate or membrane), beads or particles (e.g., latex particles), tube (e.g., test tube), or the like. Preferable examples of the solid phase when fixing the antigen as antigen for screening include a microplate. Preferable examples of the solid phase when fixing the antigen as antigen for immunoassay include a microplate and latex particles.

The antibody according to the present invention may be easily produced by dissolving the antigen in a solvent such as phosphate buffered saline (PBS), and administering the solution to an animal to effect immunization. An appropriate adjuvant may optionally be added to the solution, and the animal may be immunized using the resulting emulsion. As an adjuvant, water-in-oil emulsion, water-in-oil-in-water emulsion, oil-in-water emulsion, liposomes, aluminum hydroxide gels, proteins or peptidic substances derived from biological substances, or the like may be used. For example, a Freund's incomplete adjuvant, a Freund's complete adjuvant, or the like may be suitably used. Administration route, dosage, and dosage timing of the adjuvant are not particularly limited, but are appropriately selected so that desired immune responses in the animal that is immunized with the antigen can be enhanced.

The type of animal that is immunized is not particularly limited, but is preferably a mammal. Examples of the mammal include mouse, rat, cattle, rabbit, goat, sheep, and the like. Among these, mouse is preferably used. The animal may be immunized by a method that is available in the field. For example, the animal may be immunized by subcutaneously, intracutaneously, intravenously, or intraperitoneally injecting an antigen solution (preferably a mixture with an adjuvant) into the animal. The immune response generally differs depending on the type and the line of animals to be immunized. Therefore, it is desirable to appropriately set the immunization schedule depending on the animal used. It is preferable to repeatedly administer the antigen several times after the initial immunization.

When obtaining a polyclonal antibody according to the present invention, the antibody may be obtained from serum (antiserum) of the immunized animal. In this case, any method that may be utilized by a person having ordinary skill in the art may be used. The antibody may be purified by appropriately combining DEAE anion exchange chromatography, affinity chromatography using protein A or the like, ammonium sulfate fractionation, PEG fractionation, ethanol fractionation, and the like. It may be easily determined by well-known methods whether or not the resulting antibody is

the antibody according to the present invention (i.e., whether or not the resulting antibody recognizes the compound shown by the formula (I), but weakly reacts with or substantially does not recognize other new quinolone antibiotics or a drug that is used concurrently with levofloxacin (e.g., antibiotic, antiphlogistic analgesic, combination cold drug, airway mucosa adjusting/normalizing agent, and bronchodilator)). Animal immunization methods, antibody purification methods, and antibody characterization methods used in connection with the method of producing the antibody according to the present invention are described in the examples described later. A person having ordinary skill in the art would easily produce the antibody according to the present invention referring to the above general description and the specific methods described in the examples while appropriately modifying or altering these methods, as required.

Examples of the new quinolone antibiotics other than the compound shown by the formula (I) include analogues of the compound shown by the formula (I). Specific examples of the new quinolone antibiotics other than the compound shown by the formula (I) include ciprofloxacin, tosufloxacin, gatifloxacin, sparfloxacin, fleroxacin, lomefloxacin, enoxacin, moxifloxacin, pazufloxacin, and the like.

Examples of the drug that is used concurrently with the compound shown by the formula (I) include diclofenac sodium, nabumetone, flurbiprofen, ketoprofen, loxoprofen sodium, oxaprozin, naproxen, ibuprofen, carbocisteine, salicylamide, acetaminophen, anhydrous caffeine, methylenedisalicylic acid, promethazine, and theophylline.

The antibody according to the present invention may be an antibody that does not cross-react with or weakly cross-reacts with any one of these compounds. When using the antibody to measure the compound shown by the formula (I), the compound can be specifically measured even if the above compounds are present in the sample.

The procedure described below is performed when obtaining a monoclonal antibody. Note that the procedure is not limited thereto. A method of producing a monoclonal antibody is well-known and is widely used in the field. Therefore, a person having ordinary skill in the art would easily produce the antibody according to the present invention using the above antigen (refer to Antibodies, A Laboratory Manual (Cold Spring Harbor Laboratory Press, (1988), chapter 6, for example).

After final immunization, the spleen cells or the lymph node cells (i.e., antibody-producing cells) are removed from the immunized animal, and fused with myeloma-derived cell lines having high growth capacity to produce a hybridoma. It is preferable to use cells having high antibody producibility (quality and amount) for cell fusion. It is preferable that the myeloma-derived cell lines be compatible with the animal from which the antibody-producing cells are derived. The cells may be fused by known methods. For example, the polyethylene glycol method, methods that utilizes Sendai virus, methods that utilizes current, or the like may be employed. The resulting hybridoma may be grown by a method that is generally used in the field. The desired hybridoma may be selected while checking the properties of the antibody produced. The hybridoma may be cloned by well-known methods such as the limiting dilution method or the soft agar method.

The hybridoma may be efficiently selected taking account of the conditions where the antibody to be produced is used for actual measurement. For example, the hybridoma that produces the desired antibody can be more efficiently selected by reacting the antibody (obtained by immunizing an animal) with the compound shown by the formula (I) that is immobilized on a solid phase in the presence of a compound for which it is desired to determine cross-reactivity, and comparing the resulting reactivity with the reactivity in the absence of the compound for which it is desired to determine cross-reactivity. A hybridoma that produces a desired antibody can also be more efficiently selected by reacting an antibody (obtained by immunizing an animal) with the compound shown by the formula (I) that is immobilized on a solid phase in the presence of a biological sample-derived component, and comparing the resulting reactivity with the reactivity in the absence of the biological sample-derived component.

After cloning steps, the binding capacity of the antibody produced with the compound shown by the formula (I) is assayed by ELISA, RIA, immunofluorescence, or the like to determine whether the selected hybridoma produces a monoclonal antibody that has the desired properties. In order to efficiently obtain an antibody that recognizes the compound shown by the formula (I), but does not recognize metabolites thereof, it is preferable to use an antibody that is obtained by binding the compound shown by the formula (I) to a protein as antigen for screening hybridoma. It is more preferable to use a protein that differs from the protein used for antigen for immunization.

A monoclonal antibody that has the desired properties may be produced by mass culture of the hybridoma that is thus selected. The mass culture method is not particularly limited. For example, a hybridoma may be cultured in an appropriate medium to produce a monoclonal antibody in the medium, or the hybridoma may be intraperitoneally injected into a mammal, and grown to produce the antibody in abdominal dropsy. The monoclonal antibody may be purified by appropriately combining the purification methods that are mentioned above in connection with the purification of antibody from antiserum, such as DEAE anion exchange chromatography, affinity chromatography, ammonium sulfate fractionation, PEG fractionation, and ethanol fractionation.

A fragment of the antibody that has antigen-antibody reactivity may also be used as the antibody according to the present invention instead of the entire antibody molecule. For example, fragments obtained by immunizing an animal, fragments obtained by recombinant DNA techniques, or chimeric antibodies may be used. A functional fragment is preferably used as the antibody fragment. Examples of the functional fragment include $F(ab')_2$, Fab', and the like. These

fragments may be produced by treating the antibody obtained as described above with proteases (e.g., pepsin or papain).

A monoclonal antibody according to the present invention may be used as an immobilized (solid-phase) antibody that is immobilized on an insoluble support, or may be used as a labeled antibody that is labeled with a well-known marker (described later). Such an immobilized antibody and a labeled antibody are also included within the scope of the present invention. For example, an immobilized antibody may be produced by causing a monoclonal antibody to be physically adsorbed on or chemically bound to an insoluble support (through an appropriate spacer, as required). The insoluble support may be formed of polymer materials (e.g., polystyrene resin), inorganic materials (e.g., glass), polysaccharide materials (e.g., cellulose or agarose), or the like. The shape of the insoluble support is not particularly limited. The insoluble support may be in the shape of plate (e.g., microplate or membrane), beads or particles (e.g., latex particles), tube (e.g., test tube), or the like.

The amount of the antibody according to the present invention that is bound to the compound shown by the formula (I) can be measured using a labeled antibody (secondary antibody) that can bind to the antibody according to the present invention. The compound shown by the formula (I) in a sample can thus be detected. Examples of the marker used to produce the labeled antibody include enzymes, fluorescent substances, chemiluminescent substances, biotin, avidin, radioactive isotopes, gold colloid particles, pigmented latex, and the like. The marker and the antibody may be bound by known methods such as the glutaraldehyde method, the maleimide method, the pyridyl disulfide method, or the periodic acid method. Note that the type and the production method of the immobilized antibody and the labeled antibody are not limited to the above examples. For example, when using an enzyme (e.g., horseradish peroxidase (HRP) or alkaline phosphatase (ALP)) as a marker, the enzyme activity can be measured using a specific substrate of the enzyme (e.g., O-phenylenediamine (OPD) or 3,3',5,5'-tetramethylbenzidine (TMB) when the enzyme is HRP, and p-nitrophenyl phosphate when the enzyme is ALP). When using biotin as a marker, at least avidin or enzyme-linked avidin is generally reacted.

The compound shown by the formula (I) that is present in a sample can be detected using an antibody according to the present invention, for example. The expression "compound shown by the formula (I)" used herein refers to the S-isomer of the compound shown by the formula (I), the R-isomer of the compound shown by the formula (I), and a racemic body of the compound shown by the formula (I) that is a 1:1 mixture of the S-isomer and the R-isomer. Therefore, the expression "antibody that reacts with the compound shown by the formula (I)" used herein refers to antibodies that react with the R-isomer of the compound shown by the formula (I), antibodies that react with the S-isomer of the compound shown by the formula (I), and antibodies that react with the S-isomer and the R-isomer of the compound shown by the formula (I).

The term "racemic body" is used synonymously with the term "ofloxacin", and the term "S-isomer of the compound shown by the formula (I)" is used synonymously with the term "levofloxacin".

The term "insoluble support" may be referred to as "solid phase", and procedures of causing antigen or antibody to be physically or chemically supported on the insoluble support, or a state in which antigen or antibody is physically or chemically supported on the insoluble support may be referred to as "immobilization" or "immobilized". The term "detection" or "measurement" should be interpreted in the broadest sense, including demonstrating the presence of and/or quantitatively determining the compound shown by the formula (I), and should not be interpreted in a narrow sense.

Examples of the detection target sample in a measurement method that utilizes an antibody according to the present invention include body fluids derived from a living body (living being). Specific examples of the detection target sample include, but are not limited to, blood, serum, plasma, urine, saliva, sputum, lacrimal fluid, otorrhea, and prostatic fluid. For example, samples that are extracted from tissues of livestock or aquatic animals, feed or water used to breed livestock or aquatic animals, or the like fall within the scope of the sample according to the present invention. Among these, body fluids of a patient who has been administered ofloxacin is particularly desirably used from the viewpoint of the relationship with treatment, etc.

The term "biological sample-derived component" used herein refers to a component that forms the sample. For example, when the biological sample is serum, the term "biological sample-derived component" refers to serum proteins (e.g., albumin or globulin), glycoproteins, lipoproteins, or the like. When the biological sample is plasma, the term "biological sample-derived component" refers to plasma proteins (including a blood coagulation factor that is not included in serum). When the biological sample is saliva, the term "biological sample-derived component" refers to enzymes (e.g., lysozyme), mucopolysaccharides, or the like. The above components may bind to ofloxacin that has been administered, for example, and may hinder the antibody from recognizing the antigenic determinant, as described in the specification.

The expression "has lost antibacterial activity" used herein refers to a state in which an antibiotic compound has changed to metabolites, digests, or the like, so that part of the antibiotic spectrum or the antibiotic activity of the unaltered compound has been lost or weakened. The term "bound" used herein refers to a state in which an antigen reversibly or irreversibly binds to sample-derived proteins in the measurement system, or is artificially bound to carriers. The term "free" used herein refers to a state in which the antigen does not bind to sample-derived proteins or carriers.

The term "recognizes" an antigen is synonymous with the term "reacts with", "cross-reacts with", or "binds to" an antigen. Note that the meaning of the term "recognizes" is not limited thereto. This term should be interpreted in the

broadest sense.

The expression "antibody according to the present invention does not cross-react with a compound," means that the antibody according to the present invention does not react with the compound. The expression "antibody according to the present invention does not cross-react with a compound" quantitatively refers to a case where cross-reactivity is less than 1% in accordance with the reaction criteria for competitive ELISA in Example 1.

The expression "antibody according to the present invention weakly cross-reacts with a compound" means that the antibody according to the present invention reacts with the compound, but has a cross-reactivity with the compound lower than that with other compounds (i.e., other compounds can be sufficiently distinguished and recognized). The expression "antibody according to the present invention weakly cross-reacts with a compound" quantitatively refers to a case where cross-reactivity is 1% or more and less than 40% in accordance with the reaction criteria for competitive ELISA in Example 1.

The expression "strongly reacts with the S-isomer" means that the antibody strongly reacts with the S-isomer of the compound shown by the formula (I) as compared with the R-isomer of the compound shown by the formula (I). The expression "strongly reacts with the S-isomer" quantitatively refers to a case where the cross-reactivity is less than 100% in accordance with the ofloxacin-antibody cross-reactivity criteria for competitive ELISA in Example 1. The expression "strongly reacts with the R-isomer" refers to a case where the cross-reactivity is more than 100% in accordance with the ofloxacin-antibody cross-reactivity criteria for competitive ELISA in Example 1.

The expression "reactivity of an antibody with the compound shown by the formula (I) does not change due to the presence of biological sample-derived components" means that substantial changes in reactivity is not observed when reacting the antibody according to the present invention with the free compound shown by the formula (I). The reactivity with the compound shown by the formula (I) may change when pharmacokinetic binding to a drug has occurred, or when a viscous substance (e.g., mucopolysaccharide contained in saliva) hinders the antibody from binding to the antigenic determinant contained in the compound shown by the formula (I), for example.

The expression "reactivity of an antibody with the compound shown by the formula (I) does not change due to binding between the compound shown by the formula (I) and serum components" refers to a property required when using the antibody according to the present invention to measure the compound shown by the formula (I) in serum samples. The above expression means that the reactivity of the antibody with the compound shown by the formula (I) is not lost or weakened even if serum components (e.g., serum albumin) are bound to the compound shown by the formula (I). The above expression quantitatively refers to a case where the difference between the reactivity at an incubation time of 0 minutes and the reactivity at an incubation time of 15 minutes is less than 5% in the test that determines the effect of incubation of the compound shown by the formula (I) with human serum by competitive ELISA in Example 3.

The condition "reactivity of an antibody with the compound shown by the formular (I) does not change due to binding between the compound and serum components" is also satisfied when the reactivity of the antibody according to the present invention with the free compound shown by the formula (I) by competitive ELISA differs less than 5% among the cases of preparing the free compound in the presence of or absence of serum.

The compound shown by the formula (I) in a biological sample may be detected using an antibody according to the present invention by known methods (e.g., Rinsho Byori, extra edition, No. 53, "immunoassay for clinical examination - technology and application-", Japanese Society of Laboratory Medicine, 1983, Eiji Ishikawa et al. (editor), "Enzyme Immunoassay", third edition, Igaku-Shoin, Ltd., 1987, and Tsunehiro Kitagawa et al. (editor), Protein, Nucleic Acid and Enzyme, separate volume, No. 31, "Enzyme Immunoassay", Kyoritsu Shuppan, Co., Ltd., 1987). Note that the method of detecting the compound shown by the formula (I) using an antibody according to the present invention is not limited to the above methods. A person having ordinary skill in the art would appropriately select the method depending on the objective. A specific measurement method is described in the examples of the present application. A person having ordinary skill in the art would easily and reliably detect the compound shown by the formula (I) contained in biological samples referring to the methods described in the examples while appropriately modifying or altering the methods, as required.

An assay reagent (kit) according to the present invention may be roughly classified into (1) an assay reagent used when the compound shown by the formula (I) is immobilized, (2) an assay reagent used when an antibody (antibody according to the present invention) that recognizes the compound shown by the formula (I) is immobilized, and (3) an assay reagent used when the compound shown by the formula (I) and the antibody that recognizes the compound shown by the formula (I) are immobilized. Labeled immunoassay and particle agglutination inhibition immunoassay are described below taking ELISA (typical labeled immunoassay) and latex agglutination inhibition immunoassay (typical particle agglutination inhibition immunoassay) as examples. The following labeled immunoassay and particle agglutination inhibition immunoassay utilize competitive reactions with ofloxacin in the sample.

<Labeled immunoassay: ELISA>

(1) When the compound shown by the formula (I) is immobilized, the assay reagent (kit) may be produced using at least (a) an insoluble support on which the compound shown by the formula (I) is immobilized, and (b) an antibody that recognizes the compound shown by the formula (I). (a) The insoluble support on which the compound shown by the formula (I) is immobilized, may be obtained by immobilizing the compound shown by the formula (I) through a carrier, or introducing inter-binding functional groups into the insoluble support and the compound shown by the formula (I), and allowing the insoluble support and the compound shown by the formula (I) to react to immobilize the compound shown by the formula (I) on the insoluble support, for example. The term "carrier" used herein refers to a carrier that is interposed to immobilize the compound shown by the formula (I) on the insoluble support, and may be proteins. Note that the carrier used to immobilize the compound shown by the formula (I) on the insoluble support need not contribute to antibody production using a low-molecular-weight antigen (hapten), differing from the carrier used for antigen for immunization. Therefore, the compound shown by the formular (I) that includes the carrier may be used as antigen for immunization, and a protein or a synthetic polymer that does not have immunogenicity may be used as the carrier. (b) The antibody that recognizes the compound shown by the formula (I) may or may not be detectably labeled. When the antibody is not detectably labeled, a secondary antibody, etc. that is detectably labeled is used. When the antibody is detectably labeled, detection methods that are appropriate for the label may be used. When the detectable label is an enzyme, the assay reagent (kit) may further include enzyme reaction substrates. A preferable combination of an enzyme and its substrate, etc. have been described above.

(2) When an antibody that recognizes the compound shown by the formula (I) is immobilized, the assay reagent (kit) may be produced using at least (a) an insoluble support on which the antibody that recognizes the compound shown by the formula (I) is immobilized, and (b) the compound shown by the formula (I) that is labeled. (a) The insoluble support on which the antibody that recognizes the compound shown by the formula (I) is immobilized may be obtained by physically or chemically immobilizing the antibody. (b) The compound shown by the formula (I) that is labeled may be obtained by well-known methods. When the compound is detectably labeled, detection methods that are appropriate for the label may be used. When the detectable label is an enzyme, the assay reagent (kit) may further include enzyme reaction substrates. This is the same as in case (1).

(3) When both the compound shown by the formula (I) and an antibody that recognizes the compound shown by the formula (I) are immobilized, the assay reagent (kit) may be produced using at least (a) an insoluble support on which the compound shown by the formula (I) is immobilized, and (b) an insoluble support on which the antibody that recognizes the compound shown by the formula (I) is immobilized. The description given in (1) applies to the insoluble support (a) on which the compound shown by the formula (I) is immobilized, and the description given in (2) applies to the insoluble support (b) on which the antibody that recognizes the compound shown by the formula (I) is immobilized.

<Particle agglutination inhibition immunoassay: latex agglutination inhibition immunoassay>

Examples of an assay reagent (kit) that is used to detect the compound shown by the formula (I) contained in a sample by methods other than labeled immunoassay include (1A) an assay reagent (kit) that includes at least (a) an antibody that recognizes the compound shown by the formula (I), and (b) an immobilized synthetic polyvalent antigen, (2A) an assay reagent (kit) that includes at least (a) latex particles on which an antibody that recognizes the compound shown by the formula (I) is immobilized, and (b) a synthetic polyvalent antigen, and (3A) an assay reagent (kit) that includes at least (a) latex particles on which an antibody that recognizes the compound shown by the formula (I) is immobilized, and (b) an immobilized synthetic polyvalent antigen. The definitions of the terms "synthetic polyvalent antigen" and "immobilized synthetic polyvalent antigen" are described later.
These assay reagents (kits) may be suitably used for latex agglutination inhibition immunoassay. The particle diameter and the type of the latex particles for (a) or (b) above may be appropriately selected to obtain the desired performance (e.g., improved sensitivity). Any latex material that is appropriate for immobilization of an antigen or an antibody may be used. Examples of latex material include a latex that contains polystyrene as the main component, styrene-butadiene copolymers, (meth)acrylate polymers, and the like. Note that particles formed of metal colloid, gelatin, liposome, microcapsules, silica, alumina, carbon black, metal compounds, metal, ceramics, magnetic materials, or the like may be used instead of latex particles.
Examples of another assay reagent (kit) that is used to detect the compound shown by the formula (I) contained in a sample based on the immunochromatography measurement principle include an assay reagent (kit) that includes

at least (a) an insoluble support on which an antibody that recognizes the compound shown by the formula (I) is immobilized, and (b) the compound shown by the formula (I) that is labeled, and an assay reagent (kit) that includes at least (b) an antibody that recognizes the compound shown by the formula (I) that is labeled, and (b) the compound shown by the formula (I) or a synthetic polyvalent antigen.

The term "synthetic polyvalent antigen" refers to a polyvalent antigen (agglutinin) produced by polymerizing hapten in order to improve the degree of agglutination in low-molecular-weight antigen (hapten) immunoassay (particularly particle agglutination immunoassay). The synthetic polyvalent antigen is similar to a polyhapten, etc. The production method and the configuration of the synthetic polyvalent antigen are not particularly limited insofar as the synthetic polyvalent antigen functions as an agglutinin after polymerizing ofloxacin. A product obtained by polymerizing ofloxacin through an appropriate carrier (e.g., protein, polyamino acid, peptide, polysaccharide (low-molecular-weight polysaccharide or high-molecular-weight polysaccharide), water-soluble synthetic polymer, or spacer compound) may be used. The antigen for immunization and the antigen for screening according to the present invention also fall under the term "synthetic polyvalent antigen". The number of molecules (binding number) of the compound shown by the formula (I) that are bound to one molecule of the carrier may be adjusted in the same manner as in the method of producing antigen for immunization or antigen for screening. The binding ratio may be two or more which allows the polyvalent antigen to be recognized. The binding ratio may be appropriately selected so that the desired agglutinin performance is obtained. For example, the binding ratio in the examples described later is preferably 8 or more, and more preferably 15 or more (latex agglutination inhibition immunoassay using an assay reagent (kit) that includes (a) latex particles on which an antibody that recognizes the compound shown by the formula (I) is immobilized, and (b) a synthetic polyvalent antigen (that is produced using bovine serum albumin as a carrier)). The optimum binding ratio differs depending on the carrier type, the assay reagent, and the assay design. A person having ordinary skill in the art would produce the assay reagent after selecting the desired carrier, and determining the optimum binding ratio for each carrier.

The object and the function of the immobilized synthetic polyvalent antigen are the same as those of the synthetic polyvalent antigen. The immobilized synthetic polyvalent antigen may be a product obtained by chemically or physically binding two or more molecules of ofloxacin to an insoluble support (e.g., latex particles) (optionally through a carrier or a spacer), or a product obtained by chemically or physically binding the above synthetic polyvalent antigen to an insoluble support (e.g., latex particles).

## EXAMPLES

The present invention is further described below by way of example. Note that the present invention is not limited to the following examples.

### Example 1: Preparation of hybridoma and acquisition of antibody

(I) Materials and methods

(1) Preparation of antigen for immunization and antigen for screening

(i) 10 mg of the S-isomer (hereinafter may be referred to as "levofloxacin" or "LVFX") of the compound shown by the formula (I) was dissolved in 2 ml of a solution (0.1 mol/l phosphate buffer: 0.4 ml, DMSO: 0.2 ml, purified water: 1.4 ml) to obtain an LVFX solution. In the examples of the present application, levofloxacin hemihydrate ((-)-(S)-9-fluoro-2,3-dihydro-3-methyl-10-(4-methyl-1-piperazinyl)-7-oxo-7H-pyrido[1,2,3-de][1,4]benzoxazine-6-carboxylic acid hemihydrate) was used as the S-isomer of the compound shown by the formula (I).
(ii) 2 mg of BSA powder or 2 mg of transferrin powder was added to the LVFX solution, and dissolved in the LVFX solution with gentle stirring. The resulting solution was allowed to stand for 5 minutes in an ice bath to obtain two LVFX-protein mixtures.
(iii) 160 mg of water-soluble carbodiimide (WSC) (crosslinking agent) was added to and dissolved in each LVFX-protein mixture. The resulting solution was incubated at 4°C for 70 hours with gentle stirring while blocking light.
(iv) The solution was then dialyzed at 4°C for 2 days in PBS (pH: 7.2).
(v) The dialyzed liquid was collected. The absorbance at 330 nm was measured to determine the LVFX concentration, and the coupling rate (LVFX-protein binding ratio) was determined.

(2) Immunization and blood collection

The LVFX-BSA coupling product (LVFX-BSA) was dissolved in PBS, and mixed with an adjuvant in a ratio of

1:1. The components were mixed using a syringe to prepare an emulsion, which was used as antigen for immunization. The antigen for immunization was administered subcutaneously to three female BALB/c mice (ML-1 and ML-2: both seven weeks old, ML-3: 11 weeks old) in an amount of 10 μg (ML-1), 20 μg (ML-2), or 40 μg (ML-3). After one week, the antigen for immunization was administered subcutaneously to each mouse in the above amount again.
When 14 days had elapsed after the initiation of immunization, mouse antiserum was collected from the ocular fundus of each mouse. The antibody titre in the antiserum was determined by antigen-immobilized ELISA described later. In order to determine the reactivity of the antibody in the antiserum with free levofloxacin, the reactivity of each antiserum was determined by competitive ELISA described later in a condition in which 2 μmol/l, 10 μmol/l, or 50 μmol/l of free levofloxacin was present in the reaction system.
Normal serum collected from the ocular fundus of an unimmunized mouse was used as a control.

(3) Cell fusion

When 5 days had elapsed after collecting blood, 10 μg of LVFX-BSA was injected intravenously to the mouse ML-1 (final immunization). The spleen was removed when 3 days had elapsed after the final immunization, and cell fusion was performed by a conventional method using polyethylene glycol. Myeloma SP2/O cells were used. The resulting fused cells were suspended in a RPMI1640 medium containing hypoxanthine-aminopterin-thymidine (HAT) and 15% fetal bovine serum so that the spleen cell concentration was $2.5x10^6$/ml. The cells were dispensed into a 96-well culture plate in a volume of 0.2 ml/well. The cells were cultured at 37°C in a 5% $CO_2$ incubator.

(4) Screening

When 11 days had elapsed after cell fusion, antigen-immobilized ELISA was performed using the culture supernatant (primary screening), and wells that showed high reactivity with the LVFX-transferrin coupling product (LVFX-transferrin) were selected as primary positive wells. The cells in the primary positive well were subcultured on a 48-well plate.
When 2 days had elapsed after subculture, competitive ELISA was performed using the culture supernatant (secondary screening), and wells that showed high reactivity with free levofloxacin were selected as secondary positive wells.
The cell lines selected by secondary screening were subjected to competitive ELISA using a concurrent drug, analogues, and metabolites of levofloxacin (tertiary screening). Whether or not the reactivity of the antibody was affected by serum when human serum was present in the reaction system of competitive ELISA with free levofloxacin and when levofloxacin and serum were incubated in advance was determined using the culture supernatant of the cell lines selected by tertiary screening.

(5) Cloning and immunoglobulin (antibody) collection

Ten cell lines of hybridoma selected by tertiary screening were cloned by the limiting dilution method. In order to sample the immunoglobulin (antibody) produced by each hybridoma, 12-weeks-old female BALB/c mice, to which 0.5 ml of pristane was intraperitoneally injected two weeks earlier, were intraperitoneally administered the hybridoma ($0.5\times10^6$ cells). Abdominal dropsy was sampled after 14 days, and centrifuged. The supernatant was mixed with an equal volume of adsorption buffer (3 mol/l NaCl, 1.5 mol/l glycine-NaOH buffer, pH: 8.5). The mixture was then filtered. The filtrate was passed through a protein A column that was equilibrated using the adsorption buffer so that the antibody contained in the filtrate was adsorbed on the column, followed by elution with a 0.1 mol/l citrate buffer (pH: 3.0). The eluate was neutralized with a 1 mol/l Tris-HCl buffer (pH: 8.0), dialyzed in PBS to collect the antibody.

(6) Subclass determination

10 types of immunoglobulins (antibodies) thus collected were subjected to subclass determination using a subtyping kit (manufactured by ZYMED).

(7) Preparation of ELISA plate

LVFX-transferrin was dissolved in PBS at a concentration of 1 μg/ml, and used as antigen for screening. The antigen was immobilized on a 96-well plate in a volume of 50 μl/well, then allowed to stand at 4°C overnight. After washing the 96-well plate with PBST (0.05% Tween 20-PBS) three times (400 μl/well), a blocking reagent (3% skimmed milk-PBST) was dispensed into the 96-well plate in a volume of 100 μl/well. The 96-well plate was allowed to stand at room temperature for one hour to prepare an ELISA plate. After washing with PBST three times, each reagent was added to the ELISA plate. The ELISA plate was then used for each ELISA test described in the examples.

(8) Antigen-immobilized ELISA

(i) Each mouse antiserum that was diluted stepwise with 1% BSA-PBST by a factor of 5 in eight steps starting from 100 fold, or the culture supernatant of the fused cells was dispensed into the ELISA plate in a volume of 50 μl/well. The ELISA plate was then allowed to stand at room temperature for one hour.
(ii) After washing with PBST three times, a solution prepared by diluting HRP-GtF(ab')$_2$-Anti-Mouse Ig's (Biosource, AMI4404) with 1% BSA-PBST by a factor of 5000, was dispensed into the ELISA plate in a volume of 50 μl/well. The ELISA plate was then allowed to stand at room temperature for one hour.
(iii) After washing with PBST three times, an OPD color reagent (prepared by dissolving OPD (2 mg/ml) and hydrogen peroxide (concentration: 0.02%) in citrate buffer (pH: 5.0)) was dispensed into the ELISA plate in a volume of 50 μl/well. The ELISA plate was then allowed to stand at room temperature for 10 minutes.
(iv) The reaction was terminated by adding 0.75 mol/l sulfate (50 μl/well). The absorbance at 492 nm was then measured using a plate reader.

(9) Preparation of compound solution used for competitive ELISA

Levofloxacin, concurrent drugs of levofloxacin, new quinolone antibiotics that are analogues of levofloxacin, metabolites of levofloxacin, and ofloxacin (racemic body) were selected as compounds used for competitive ELISA. A solvent that easily dissolves each compound was selected from purified water, PBST, DMSO, methanol, 0.1 mol/l HCl, and 0.1 mol/l NaOH. When selecting 0.1 mol/l HCl or 0.1 mol/l NaOH as the solvent, a solution obtained by dissolving the compound was immediately diluted with 1% BSA-PBST by a factor of 50, and whether or not the pH of the solution was about neutral was determined using pH test paper. The solution was diluted with 1% BSA-PBST stepwise so that the concentration of the compound was 0.01 μmol/l, 0.1 μmol/l, 1 μmol/l, 10 μmol/l, or 100 μmol/l, and used for competitive ELISA. For example, levofloxacin was dissolved in purified water at a concentration of 1 mmol/l, diluted with 1% BSA-PBST stepwise, and used for competitive ELISA.

<Concurrent drugs of levofloxacin subjected to cross-reactivity test>

Diclofenac sodium, nabumetone, flurbiprofen, ketoprofen, loxoprofen sodium, oxaprozin, naproxen, ibuprofen, carbocisteine, salicylamide, acetaminophen, anhydrous caffeine, methylenedisalicylic acid, promethazine, and theophylline

<Analogues of levofloxacin subjected to cross-reactivity test>

Ciprofloxacin, tosufloxacin, gatifloxacin, sparfloxacin, fleroxacin, lomefloxacin, norfloxacin, enoxacin, moxifloxacin, and pazufloxacin
<Metabolites of levofloxacin subjected to cross-reactivity test>
N-oxide metabolite and desmethyl metabolite

<Ofloxacin>

Mixture of the S-isomer and the R-isomer of the compound shown by formula (I) (composition ratio: 1:1)

(10) Competitive ELISA

(i) Each compound solution prepared in the section entitled "(9) Preparation of compound solution used for competitive ELISA" was dispensed into the ELISA plate in a volume of 25 μl/well.
(ii) Each mouse antiserum that was diluted with 1% BSA-PBST by a factor of 1000, or the culture supernatant of the fused cells was dispensed into the ELISA plate in a volume of 25 μl/well. The ELISA plate was then allowed to stand at room temperature for one hour.

The subsequent procedures were performed in the same manner as the steps (ii) to (iv) described in the section entitled

"(8) Antigen-immobilized ELISA".

(11) Evaluation of effects of serum on competitive ELISA using the antibody according to the present invention

(i) Human serum was diluted with 1% BSA-PBST to prepare 5% human serum. Human serum and plasma used in each example were derived from the blood of volunteers that was collected with consent.
(ii) 2.0 μmol/l, 0.2 μmol/l, or 0.02 μmol/l levofloxacin was prepared using 5% human serum ("human serum reagent a"). As a control reagent, 2.0 μmol/l, 0.2 μmol/l, or 0.02 μmol/l levofloxacin was prepared using 1% BSA-PBST.
(iii) The human serum reagent a or the control reagent was dispensed into the ELISA plate in a volume of 25 μl/well.
(iv) The culture supernatant of the fused cells was dispensed into the ELISA plate in a volume of 25 μl/well. The ELISA plate was allowed to stand at room temperature for one hour.

The subsequent procedures were performed in the same manner as the steps (ii) to (iv) described in the section entitled "(8) Antigen-immobilized ELISA."

(12) Evaluation of effects of serum on competitive ELISA using samples prepared by incubating free levofloxacin and human serum

(i) A solution prepared by dissolving levofloxacin in purified water was 100-fold diluted with human serum to prepare a 30 μmol/l levofloxacin solution. The levofloxacin solution was incubated at 37°C for 0, 15, or 60 minutes.
(ii) After incubation, the 30 μmol/l levofloxacin solution was diluted with 1% BSA-PBST by a factor of 15, 150, or 1500 to prepare human serum reagent b.
(iii) The human serum reagent b was dispensed into the ELISA plate in a volume of 25 μl/well.
(iv) The culture supernatant of the fused cells was dispensed into the ELISA plate in a volume of 25 μl/well. The ELISA plate was allowed to stand at room temperature for one hour.

The subsequent procedures were performed in the same manner as the steps (ii) to (iv) described in the section entitled "(8) Antigen-immobilized ELISA".

(13) Quantification of reaction

(i) The reactivity (%) of each compound at each concentration was calculated by the following expression (12) using the measured absorbance.

$$\text{Reactivity (\%)} = [\text{Abs.}(x)] / [\text{Abs.}(0)] \times 100 \qquad (1)$$

Abs.(x):
(absorbance at compound concentration of x μmol/l) - (absorbance at antibody concentration of 0 μg/ml)
Abs.(0):
(absorbance at compound concentration of 0 μmol/l) - (absorbance at antibody concentration of 0 μg/ml)
(ii) The relationship between the concentration and the reactivity of each compound was plotted on a graph, and the concentration of each compound at which the reactivity was 50% was determined. This concentration was determined to be the half maximal (50%) inhibitory concentration ($IC_{50}$).
(iii) The cross-reactivity rate (%) of each antibody with each compound was calculated by the following expression using the concentration $IC_{50}$. Criteria for the cross-reactivity of each antibody with each compound was set based on the cross-reactivity rate thus calculated.

$$\text{Cross-reactivity rate (\%)} = [IC_{50} \text{ of levofloxacin}] / [IC_{50} \text{ of each compound}] \times 100 \quad (2)$$

High: 80 to 100%
Middle: 40% or more and less than 80%
Low: 1% or more and less than 40%
None: 0% or more and less than 1%
Criteria for the cross-reactivity of each antibody with ofloxacin were set as follows.
Strongly reacted with the S-isomer: <100%
Equally reacted with the the S-isomer and the R-isomer: 100%
Strongly reacted with the R-isomer: >100%

(II) Results

(1) Preparation of antigen for immunization and antigen for screening

The LVFX-protein binding ratios for antigen for immunization and antigen for screening were high (LVFX-BSA: 12.0, LVFX-transferrin: 13.9). The binding ratio was calculated by dividing the concentration of LVFX after dialysis by the concentration of BSA or transferrin before dialysis, and indicates the number of LVFX molecules bound to one molecule of BSA or transferrin.

(2) Results of antigen-immobilized ELISA test (blood collection)

As a result of blood collection and determination of the antibody titre in each mouse antiserum by antigen-immobilized ELISA, a high antibody titre was obtained in all of the three mouse antisera (FIG. 1). Since a protein coupled to an antigen for screening that was immobilized on the ELISA plate differed from a protein coupled to an antigen for immunization, there was no possibility that each antibody non-specifically reacted with the protein site (carrier) of the immobilized antigen for screening. Therefore, it is considered that each mouse antiserum obtained in this example included an antibody that specifically recognized levofloxacin that was common to the antigen for immunization and the antigen for screening.

(3) Results of competitive ELISA test (blood collection)

Free levofloxacin that was not bound to the protein was allowed to present (compete with the immobilized antigen) in the competitive ELISA reaction system, and a change in reactions between the antigen for screening and the antibody in each mouse antiserum was determined. As a result, the reactions between the antigen for screening and the antibody decreased depending on the amount of levofloxacin added to the reaction system when using each mouse antiserum (FIG. 2). Therefore, it is considered that each mouse antiserum obtained in this example included an antibody that recognized free levofloxacin.

(4) Screening

As a result of primary screening, 63 cell lines that showed high reactivity with immobilized LVFX-transferrin were selected as primary positive cell lines. As a result of secondary screening, 32 cell lines that showed high reactivity with free levofloxacin were selected as secondary positive cell lines. As a result of tertiary screening, 10 cell lines that did not cross-react with, or weakly cross-reacted with the concurrent drugs, the analogues, and the metabolites of levofloxacin were selected, and the antibodies derived from these 10 cell lines were tested.

Human serum was allowed to present in the competitive ELISA reaction system, and the effects of the serum component on each of the ten antibodies selected by tertiary screening were determined. In this case, free levofloxacin may bind to serum proteins to form bound levofloxacin due to the addition of the serum, so that the reaction of the antibody may be affected. However, the difference in reactivity between the control (serum was not added) and the case where the serum was added was less than 5% for each antibody.

Each of the ten antibodies selected by tertiary screening was subjected to competitive ELISA using the human serum reagent b that was prepared by incubating free ofloxacin and serum so that free ofloxacin was converted into

bound ofloxacin that was bound to serum proteins. The reactivity of the antibody was determined by competitive ELISA. As a result, the difference between the reactivity at an incubation time of 0 minutes and the reactivity at an incubation time of 15 or 60 minutes was less than 5%.

From the above two test results for the effects of serum, each of the ten antibodies selected by tertiary screening showed the same reactivity with bound levofloxacin that was bound to serum proteins as the reactivity with free levofloxacin that was not bound to serum proteins. Therefore, it is estimated that free levofloxacin and bound levofloxacin in the sample can be detected at the same time.

(5) Cloning, immunoglobulin (antibody), and subclass determination

The subclass of each antibody selected by tertiary screening was determined. The subclass of eight antibodies among the ten antibodies was IgG, and the subclass of the remaining two antibodies was IgA or IgM. Only the eight monoclonal antibodies 77201 to 77207 and 77209 (subclass: IgG) were subsequently evaluated.
Hybridomas that produce seven monoclonal antibodies among the eight monoclonal antibodies excluding the monoclonal antibody 77203 that showed reactivity with desmethyl levofloxacin to some extent in Example 2 were deposited with the National Institute of Advanced Industrial Science and Technology (AIST Tsukuba Center 6, 1-1, Higashi 1-chome, Tsukuba-shi, Ibaraki-ken, Japan). The deposit numbers are as follows.
Antibody number: deposit number
77201: FERM BP-11010
77202: PERM BP-11011
77204: FERM BP-11012
77205: FERM BP-11013
77206: FERM BP-11014
77207: FERM BP-11015
77209: FERM BP-11016

Example 2

Evaluation of cross-reactivity of monoclonal antibody produced by each hybridoma

(I) Materials and methods

(1) Preparation of reagent

Each compound used for competitive ELISA was prepared in the same manner as in Example 1(I)(9).

(2) Evaluation of cross-reactivity (competitive ELISA)

Competitive ELISA was performed in the same manner as in Example 1(I)(10). The seven monoclonal antibodies selected in Example 1(II)(5) (purified IgG: 0.2 μg/ml) were used. The reactivity and the cross-reactivity of each antibody were quantified in the same manner as in Example 1(I)(13).

(II) Results

(1) Reactivity of each monoclonal antibody with free levofloxacin

As shown in FIG. 3, each antibody showed reactivity with free levofloxacin.

(2) Cross-reactivity with analogues, metabolites, the S-isomer (levofloxacin), the R-isomer, and racemic body (ofloxacin) (1:1 mixture of the S-isomer and the R-isomer) of the compound shown by formula (I)

Table 1 shows the cross-reactivity rate of each antibody with the analogues, the levofloxacin metabolites, and ofloxacin.

The monoclonal antibodies 77201, 77202, and 77204 showed cross-reactivity with moxifloxacin. The monoclonal antibodies 77205, 77207, and 77209 showed some cross-reactivity with fleroxacin, but showed no or only weak cross-reactivity with other analogues.

The monoclonal antibodies 77201, 77202, 77204, 77205, and 77207 showed equal cross-reactivity with the S-isomer and ofloxacin. The monoclonal antibody 77206 showed cross-reactivity with ofloxacin of 50%. Therefore, it

was found that the monoclonal antibody 77206 was an antibody that strongly reacts with the S-isomer. The monoclonal antibody 77209 showed cross-reactivity with ofloxacin of 250% (i.e., showed high cross-reactivity with ofloxacin as compared with levofloxacin). Therefore, it was found that the monoclonal antibody 77209 was an antibody that strongly reacts with the R-isomer.

The cross-reactivity of each antibody with the levofloxacin metabolites and ofloxacin is summarized below.
77201, 77202, 77204, 77205, and 77207: The antibodies 77201, 77202, 77204, 77205, and 77207 react with ofloxacin, but do not react with the N-oxide metabolite and the desmethyl metabolite thereof. The antibodies 77201, 77202, 77204, 77205, and 77207 react with the S-isomer (levofloxacin) and the R-isomer of the compound shown by the formula (I).
77206: The antibody 77206 reacts with ofloxacin, but do not react with the N-oxide metabolite and the desmethyl metabolite thereof. The antibody 77206 strongly reacts with levofloxacin.
77209: The antibody 77209 reacts with ofloxacin, but do not react with the N-oxide metabolite and the desmethyl metabolite thereof. The antibody 77209 strongly reacts with the R-isomer of ofloxacin.

[TABLE 1]

| Antibody No. | | Analogue | | | | | | | | | | Metabolite | | Racemic body |
|---|---|---|---|---|---|---|---|---|---|---|---|---|---|---|
| | Levofloxacin | Ciprofloxacin | Tosufloxacin | Gatifloxacin | Sparfloxacin | Fleroxacin | Lomefloxacin | Norfloxacin | Enoxacin | Moxifloxacin | Pazufloxacin | Desmethyl metabolite | N-Oxide metabolite | Ofloxacin |
| 77201 | 100 | ± | ± | | | 30 | ± | ± | ± | 60 | | ± | | 100 |
| 77202 | 100 | ± | | ± | | 25 | ± | ± | ± | 71 | | | | 100 |
| 77204 | 100 | ± | | | | 35 | ± | ± | ± | 70 | | | | 100 |
| 77205 | 100 | | | | | 33 | | 1.1 | 1.3 | | | | | 100 |
| 77206 | 100 | | | | | 5 | | | | | | ± | | 50 |
| 77207 | 100 | ± | | | | 20 | | ± | | 5.7 | | | | 100 |
| 77209 | 100 | ± | | | | 33 | | ± | 1 | ± | | | | 250 |

The blank column indicates that the cross-reactivity rate was less than 0.1%, and the symbol "±" indicates that the cross-reactivity rate was 0.1% or more and less than 1%.

The cross-reactivity with the typical levofloxacin concurrent drugs was less than 0.1 %. Therefore, it was determined that each antibody does not react with the typical levofloxacin concurrent drugs.

Example 3

Evaluation of effects of serum (competitive ELISA)

(I) Materials and methods

(1) Effects of addition of serum

The same procedures as those described in "(11) Evaluation of effects of serum on competitive ELISA using the antibody according to the present invention" in Example 1(I) were performed, except for changing the culture supernatant of the fused cells in the step (iv) to a monoclonal antibody (purified IgG, 0.2 μg/mg). The reactivity and the cross-reactivity of each antibody were quantified in the same manner as in Example 1(I)(13).

(2) Effects of serum when incubating free levofloxacin and human serum in advance

The same procedures as those described in "(12) Evaluation of effects of serum on competitive ELISA using sample prepared by incubating free levofloxacin and human serum" in Example 1(I) were performed, except for changing the culture supernatant of the fused cells to a monoclonal antibody (purified IgG, 0.2 μg/mg). The reactivity and the cross-reactivity of each antibody were quantified in the same manner as in Example 1(I)(13).

(II) Results

(I) Effects of addition of serum

Human serum was allowed to present in the competitive ELISA reaction system, and the effects of the serum components on the reactivity of the antibody were determined. Free levofloxacin may bind to proteins in the serum to form bound levofloxacin due to the addition of the serum, so that the reaction of the antibody may be affected. However, the difference in reactivity between the control (serum was not added) and the case where serum was added was less than 5% for each antibody.

(2) Effects of addition of free levofloxacin and human serum incubated in advance

The human serum reagent b that was prepared by incubating free ofloxacin and serum so that free ofloxacin was converted into bound ofloxacin that was bound to serum proteins was used as the sample of competitive ELISA. The reactivity of the antibody was determined by competitive ELISA. As a result, the difference between the reactivity at an incubation time of 0 minutes and the reactivity at an incubation time of 15 or 60 minutes was less than 5%.

As is clear from the results of (1) and (2), it was found that the antibody according to the present invention was not affected by serum proteins during antigen-antibody reaction. Therefore, the antibody according to the present invention may be used to measure the drug concentration in blood.

Example 4

Immunoassay of levofloxacin in sample using the antibody according to the present invention

(I) Materials and methods

(1) Preparation of reagent

Levofloxacin powder was dissolved in purified water (1 mmol/l) to prepare a standard levofloxacin solution (standard solution). The standard solution was dispensed into cryogenic vials, and stored (frozen) at -80°C.

(2) Calibration curve

(i) The standard solution was diluted with 1% BSA-PBST in six steps (0.0625 to 2 μmol/l) to prepare standard samples.
(ii) The standard sample was dispensed into an ELISA plate in a volume of 25 μl/well. Next, the antibody 77206 (purified IgG, 0.1 μg/ml, 1% BSA-PBST) was dispensed into the ELISA plate in a volume of 25 μl/well. The ELISA plate was allowed to stand at room temperature for one hour.

The subsequent procedures were performed in the same manner as the steps (ii) to (iv) described in the section entitled "(8) Antigen-immobilized ELISA" in Example 1(I).

(3) Dilution linearity test

(i) The standard solution was diluted with human serum at appropriate concentrations to prepare three types of levofloxacin-containing human serum.
(ii) Each levofloxacin-containing human serum was diluted with 1% BSA-PBST by a factor of 11, and then 2-fold diluted up to a factor of 704 to prepare dilution linearity test samples.
(iii) The dilution linearity test samples were dispensed into the ELISA plate in a volume of 25 μl/well. Next, the antibody 77206 (purified IgG, 0.1 μg/ml, 1% BSA-PBST) was dispensed into the ELISA plate in a volume of 25 μl/well. The ELISA plate was allowed to stand at room temperature for one hour.

The subsequent procedures were performed in the same manner as the steps (ii) to (iv) described in the section entitled "(8) Antigen-immobilized ELISA." in Example 1 (I). The measured absorbance was converted into concentration using the calibration curve, and the serum dilution ratio (x-axis) (e.g., 10-fold dilution is indicated by "10/100") and the concentration conversion value (y-axis) were plotted on a graph. The correlation coefficient between the concentration conversion value and the serum dilution ratio was also calculated.

(4) Repeatability test

(i) The standard solution was diluted with 1% BSA-PBST to a concentration of 30 μmol/l, 1.0 μmol/l, or 6 μmol/l, diluted with serum by a factor of 11, and diluted with 1% BSA-PBST by a factor of 2 to prepare the repeatability test sample (samples A, B, and C).
(ii) The repeatability test sample was dispensed into an ELISA plate in a volume of 25 μl/well. Next, the antibody 77206 (purified IgG, 0.1 μg/ml, 1% BSA-PBST) was dispensed into the ELISA plate in a volume of 25 μl/well. The ELISA plate was allowed to stand at room temperature for one hour.

The subsequent procedures were performed in the same manner as the steps (ii) to (iv) described in the section entitled "(8) Antigen-immobilized ELISA" in Example 1(I). The measured absorbance was converted into concentration using the calibration curve. Each repeatability test sample was measured eight times, and the average value, standard deviation, and coefficient of variation of the measured values were calculated.

(5) Recovery test

(i) The standard solution was diluted with 1% BSA-PBST at an appropriate concentration, diluted with serum by a factor of 11, and further diluted with 1% BSA-PBST by a factor of 2 to prepare a recovery test sample.
(ii) The recovery test sample was dispensed into an ELISA plate in a volume of 25 μl/well. Next, the antibody 77206 (purified IgG, 0.1 μg/ml, 1% BSA-PBST) was dispensed into the ELISA plate in a volume of 25 μl/well. The ELISA plate was allowed to stand at room temperature for one hour.

The subsequent procedures were performed in the same manner as the steps (ii) to (iv) described in the section entitled "(8) Antigen-immobilized ELISA" in Example 1(I). The measured absorbance was converted into concentration using the calibration curve, and the theoretical value (x-axis) and the concentration conversion value (y-axis) were plotted on a graph. The regression line and the correlation coefficient of the concentration conversion value and the theoretical value were also calculated.

(II) Results

(1) Calibration curve

A calibration curve was drawn using the standard sample in the range of 0.0625 to 2 μmol/l. The calibration curve showed an excellent linearity within the above range. It was thus confirmed that the measurement method according to the present invention can be used to quantitatively determine the compound shown by the formula (I) in a sample (FIG. 4).

(2) Dilution linearity test

The correlation coefficient of the concentration conversion value and the of the serum dilution ratio of each of the three dilution linearity test samples was 0.99 or more. Specifically, an excellent dilution linearity was obtained (FIG. 5). It was thus found that a measurement system that is not affected by the serum component derived from the sample can be obtained using the antibody according to the present invention.

(3) Repeatability test

Each of the three repeatability test samples was simultaneously measured eight times. The coefficient of variation (CV%) of each sample was less than 10%. Specifically, high repeatability was obtained (Table 2).

[TABLE 2]

| | LVFX(μmol/L) | | |
|---|---|---|---|
| | Sample A | Sample B | Sample C |
| 1 | 1.36 | 0.52 | 0.29 |
| 2 | 1.58 | 0.49 | 0.33 |
| 3 | 1.40 | 0.53 | 0.31 |
| 4 | 1.54 | 0.50 | 0.32 |
| 5 | 1.53 | 0.51 | 0.31 |
| 6 | 1.48 | 0.47 | 0.30 |
| 7 | 1.47 | 0.42 | 0.29 |
| 8 | 1.60 | 0.40 | 0.23 |
| mean | 1.50 | 0.48 | 0.30 |
| SD | 0.08 | 0.05 | 0.03 |
| CV(%) | 5.5 | 9.6 | 9.8 |

(4) Measurement of sample having known concentration

The regression line of the theoretical value and the concentration conversion value of the recovery test sample was y=1.078x+0.021. The correlation coefficient was 0.993 (FIG. 6). It was thus confirmed that the measurement method using the antibody according to the present invention can accurately measure the concentration of the compound shown by the formula (I) in biological samples.

Example 5

When biological sample was saliva

(I) Materials and methods

(1) Preparation of reagent

The standard solution prepared in Example 4(I)(1) was used.

(2) Competitive ELISA

(i) A 10%, 5%, or 2% diluted human saliva solution was prepared using 1% BSA-PBST.
(ii) The standard solution was diluted with each diluted human saliva solution to a concentration of 0.2 μmol/l to prepare human saliva samples. A sample that did not contain human saliva (0%) was used as a control.
(iii) The human saliva samples were dispensed into an ELISA plate in a volume of 25 μl/well.
(iv) The antibody 77201 (purified IgG, 0.2 μg/ml, 1% BSA-PBST) was dispensed into the ELISA plate in a volume of 25 μl/well. The ELISA plate was allowed to stand at room temperature for one hour.

The subsequent procedures were performed in the same manner as the steps (ii) to (iv) described in the section entitled "(8) Antigen-immobilized ELISA" in Example 1(I).

(II) Results

(1) When biological sample was saliva

The difference between the reactivity when the saliva concentration in the sample was 0% and the reactivity when the saliva concentration in the sample was 10%, 5%, or 2% was less than 5%. Specifically, the measurement was not affected by the salivary component (FIG. 7). Components (e.g., lysozyme and mucopolysaccharide) that inhibit the immune reaction system are contained in saliva, and the epitope in the free antigen may be covered with the salivary

components. However, it was confirmed that the assay using the antibody according to the present invention can accurately measure the concentration of the compound shown by the formula (I) in a biological sample without being affected by the salivary components.

Example 6

Evaluation of effects of plasma (competitive ELISA)

The effects of plasma that contains a large number of types and a large amount of proteins as compared with serum were examined.

(I) Materials and methods

(1) Preparation of reagent

Each compound used for competitive ELISA was prepared in the same manner as in Example 1(I)(9).

(2) Evaluation of effects of plasma on competitive ELISA

(i) 10% human plasma was prepared using 1% BSA-PBST.
(ii) 0.4 μmol/l, 0.2 μmol/l, or 0.01 μmol/l levofloxacin was prepared using the 10% human plasma ("human plasma reagent a"). As control reagents, 0.4 μmol/l, 0.2 μmol/l, or 0.01 μmol/l levofloxacin was prepared using 1% BSA-PBST.
(iii) The human plasma reagent a or the control reagent was dispensed into an ELISA plate in a volume of 25 μl/well.
(iv) The antibody 77206 (purified IgG, 0.2 μg/ml, 1% BSA-PBST) was dispensed into the ELISA plate. The ELISA plate was allowed to stand at room temperature for one hour.

The subsequent procedures were performed in the same manner as the steps (ii) to (iv) described in the section entitled "(8) Antigen-immobilized ELISA" in Example 1(I).

(II) Results

(1) Effects of addition of plasma

It was estimated that the free antigen binds to plasma proteins to form a bound antigen due to the addition of plasma, so that the competitive reaction is inhibited. However, the difference in reactivity between the control (plasma was not added) and the case where the plasma was added was less than 5% for each antibody (FIG. 8). Therefore, the antibody according to the present invention may be used to measure the drug concentration in blood without being affected by plasma proteins during the antigen-antibody reaction.

Comparative Example

(1) Properties of conventional antibody (effects of plasma component)

(I) Materials and methods

The method disclosed in Patent Document 2 was partly modified.

(i) Goat anti-rabbit IgG prepared to a concentration of 20 μg/ml using a 0.1 mol/l carbonate buffer (pH: 9.5) was dispensed into a 96-well plate in a volume of 100 μl/well, and incubated at room temperature for two hours to immobilize the primary antibody. The plate was washed three times with a washing agent (0.05% Tween 20, 50 mmol/l Tris-HCl, pH: 7.4).
(ii) A 50 mmol/l Tris-HCl buffer (pH: 7.4) containing 1% BSA was added to the plate in a volume of 300 μl/well, followed by incubation at room temperature for two hours. The plate was then washed three times with the washing agent.
(iii) Next, anti-levofloxacin rabbit antiserum diluted to an appropriate concentration with a 50 mmol/l Tris-HCl buffer

(EIA buffer) containing 0.5% BSA was added to the plate, followed by incubation at room temperature for two hours so that the anti-levofloxacin antibody in the anti-levofloxacin rabbit antiserum was captured by the primary antibody. The plate was then washed three times with the washing agent.
(iv) 50 μl of an alkaline phosphatase-labeled antigen diluted with an EIA buffer by a factor of 50, and 50 μl of a 10 μg/ml levofloxacin solution diluted with an EIA buffer containing 10% human plasma or an EIA buffer that did not contain human plasma was added to each well, followed by incubation at room temperature for two hours.
(v) After washing the plate three times with the washing agent, 100 μl of a p-nitrophenyl phosphate solution was added, followed by incubation at room temperature for 30 minutes. The reaction was terminated by adding 25 μl of a 1.6 mol/l sodium hydroxide solution. The absorbance at 405 nm was then measured.

(II) Results

The reactivity of the sample to which plasma was added was approximately twice the reactivity of the sample to which plasma was not added (FIG. 9). This is considered to be because free levofloxacin was bound to plasma proteins to form bound levofloxacin so that the antibody could not recognize the epitope in levofloxacin (i.e., the competitive reaction was inhibited). The antigen-antibody reaction was not affected by the enzyme-labeled antigen in the reaction solution since the labeling enzyme that was bound to the antigen inhibited binding between the plasma protein and levofloxacin.

Example 7

Another competitive ELISA (one-step competitive ELISA)

Competitive ELISA described in the section entitled "(8) Antigen-immobilized ELISA" in Example 1(I) may be performed by the following method that achieve one-step detection by labeling the antibody with HRP in advance.

(I) Materials and methods

(1) Preparation of HRP-labeled antibody

(i) The monoclonal antibodies 77201, 77202, 77204, 77205, 77206, 77207, and 77209 were dialyzed at 4°C for two days in a 0.1 mol/l sodium hydrogen carbonate buffer (pH: 9.3). After recovering the dialyzed solution, the absorbance at 280 nm was measured to confirm the antibody concentration.
(ii) 50 mg of HRP ("peso-301" manufactured by Toyobo Co., Ltd.) was dissolved in 5 ml of a 5 mmol/l acetate buffer (pH: 4.5). After the addition of 21.5 μl of a sodium periodate aqueous solution (100 mg/ml), the mixture was allowed to stand at room temperature for 30 minutes while blocking light.
(iii) The mixture was purified using a PD-10 column (Amersham Biosciences, 17-0851-01) (eluant: 5 mM acetate buffer (pH: 4.5)) to recover a dark green solution fraction. The concentration of the activated HRP contained in the solution was determined by measuring the absorbance at 280 nm.
(iv) The antibody prepared in (i) and the activated HRP prepared in (iii) were diluted with a 0.1 mol/l sodium hydrogen carbonate buffer (pH: 9.3) to a concentration of 1 mg/ml. 250 μl of each solution was mixed, and allowed to stand at room temperature for one hour while blocking light.
(v) After the addition of 10 μl of a sodium borohydride aqueous solution (2 mg/ml), the mixture was allowed to stand at room temperature for 15 minutes while blocking light.
(vi) After the addition of 510 μl of a saturated ammonium sulfate aqueous solution, the mixture was allowed to stand on ice for one hour while blocking light.
(vii) The resulting solution was centrifuged at 4°C and 10,000 rpm for 10 minutes to obtain antibody pellets.
(viii) The antibody pellets were dissolved in 500 μl of PBS (pH: 7.2), and dialyzed at 4°C for two days in PBS (pH: 7.2).
(ix) After recovering the dialyzed solution, the absorbance at 280 nm was measured to confirm the HRP-labeled antibody concentration.

(2) One-step competitive ELISA

(i) Each levofloxacin solution prepared in the same manner as in the section entitled "(9) Preparation of compound

solution used for competitive ELISA" in Example 1(I) was dispensed into an ELISA plate in a volume of 25 μl/well.
(ii) An antibody solution prepared by diluting the HRP-labeled antibody prepared in the section entitled "(1) Preparation of HRP-labeled antibody" with 1% BSA-PBST by a factor of 70 to 3500 was dispensed into the ELISA plate in a volume of 25 μl/well. The ELISA plate was allowed to stand at room temperature for one hour.

The subsequent procedures were performed in the same manner as the steps (iii) to (iv) described in the section entitled "(8) Antigen-immobilized ELISA" in Example 1(I).

(II) Results

Free levofloxacin that was not bound to proteins was allowed to present (compete with the immobilized antigen) in a one-step competitive ELISA reaction system. As a result, the reactivity of the HRP-labeled antibody with the immobilized antigen decreased depending on the amount of levofloxacin added to the reaction system when using each HRP-labeled antibody (FIG. 10). Therefore, it is considered that one-step competitive ELISA described in this example is useful as immunoassay for detecting the compound shown by the formula (I) in a sample.

Example 8

Latex agglutination inhibition immunoassay (antibody-immobilized latex)

This example corresponds to "(20) An immunoassay for detecting the compound shown by the formula (I) in a sample, comprising competitively reacting a synthetic polyvalent antigen and the compound shown by the formula (I) in the sample with the antibody according to any one of (1) to (11) that is immobilized on a solid phase", and tested latex agglutination inhibition immunoassay (antibody-immobilized latex) (i.e., particle agglutination inhibition immunoassay).

(I) Materials and methods

(1) Preparation of reagent

(i) LVFX-BSA (binding ratio: 18) that was prepared by the method described in the section entitled "(1) Preparation of antigen for immunization and antigen for screening" in Example 1(I) was diluted with a 20 mmol/l Tris buffer (pH: 7.0, 500 mmol/l sodium chloride, 1% BSA) to a concentration of 1.0 μg/ml to obtain a first reagent. The first reagent includes "the antibody according to any one of (1) to (11) that is immobilized on a solid phase" recited in (20).
(ii) 1.5 ml of a 1% latex (manufactured by Sekisui Chemical Co., Ltd., average particle diameter: 200 nm) suspension was added to 1.5 ml of a 20 mmol/l Tris buffer (pH: 8.5) containing 0.8 mg/ml of the monoclonal antibody 77209, and the mixture was stirred at 4°C for two hours. After the addition of 3.0 ml of a 20 mmol/l Tris buffer (pH: 8.5) containing 0.1% BSA, the mixture was stirred at 4°C for one hour. After centrifuging the mixture at 4°C and 13,000 rpm for 35 minutes, the supernatant was removed, and suspended in a 5 mmol/l MOPS buffer (pH: 7.0, 0.1% BSA) in an equal volume. After ultrasonic dispersion (Nissei Ultrasonic Generator), the resulting solution was heated at 50°C for one hour. After cooling the solution, the solution was diluted with a 5 mmol/l MOPS buffer (pH: 7.0) so that the absorbance at 600 nm was 3 Abs to obtain a second reagent.
(iii) A levofloxacin solution having a concentration of 0.0 μg/ml, 1.0 μg/ml, 2.5 μg/ml, 5.0 μg/ml, 10 μg/ml, or 20 μg/ml was prepared in the same manner as in the section entitled "(9) Preparation of compound solution used for competitive ELISA" in Example 1(I). A concentrated levofloxacin standard solution having a concentration of 0.0 μg/ml, 10 μg/ml, 25 μg/ml, 50 μg/ml, 100 μg/ml, 150 μg/ml, or 200 μg/ml was prepared in the same manner as in the section entitled "(9) Preparation of compound solution used for competitive ELISA" in Example 1(I). The concentrated levofloxacin standard solution was 10-fold diluted with human serum, human plasma, or human saliva to prepare levofloxacin-containing human serum, human plasma, or human saliva having a levofloxacin concentration of 0.0 μg/ml, 1.0 μg/ml, 2.5 μg/ml, 5.0 μg/ml, 10 μg/ml, 15 μg/ml, or 20 μg/ml.

(2) Measurement procedure

Each levofloxacin solution was subjected to the measurement using a 7170S automated analyzer (manufactured by Hitachi, Ltd.). Specifically, 150 μl of the first reagent was added to 2.5 μl of each sample solution. The mixture was heated at 37°C for 5 minutes. After the addition of 150 μl of the second reagent, a change in absorbance (600 nm) at 19 to 34 photometric points was measured at 37°C.

(II) Results

The levofloxacin aqueous solution was subjected to the measurement, and a change in absorbance corresponding to each levofloxacin concentration was plotted (FIG. 11). The absorbance decreased depending on the amount of levofloxacin added to the reaction system. A calibration curve was drawn using a spline function. The levofloxacin-containing human serum or human plasma at each concentration was subjected to the measurement, and the measured value was calculated from the calibration curve. The theoretical value and the measured value of the levofloxacin solution concentration showed good correlation within the measurement range (FIGS. 12 and 13). The levofloxacin-containing saliva was subjected to the measurement, and a change in absorbance corresponding to each levofloxacin concentration was plotted. The absorbance decreased depending on the amount of levofloxacin added to the reaction system (FIG. 14).
It was thus confirmed that the latex agglutination inhibition immunoassay using an antibody-immobilized latex described in this example can be used to quantitatively determine the compound shown by the formula (I) in a serum, plasma, or saliva sample.

Example 9

Latex agglutination inhibition immunoassay (antigen-immobilized latex)

This example corresponds to "(21) An immunoassay for detecting the compound shown by the formula (I) in a sample, comprising competitively reacting an immobilized synthetic polyvalent antigen and the compound shown by the formula (I) in the sample with the antibody according to any one of (1) to (11)", and tested latex agglutination inhibition immunoassay (antigen-immobilized latex) (i.e., particle agglutination inhibition immunoassay).

(I) Materials and methods

(1) Preparation of reagent

(i) The monoclonal antibody 77206 was diluted with a 5 mmol/l MOPS buffer (pH: 7.4) to a concentration of 5.2 mg/ml to obtain a first reagent.
(ii) 3.0 ml of a 1% latex (manufactured by Sekisui Chemical Co., Ltd., average particle diameter: 210 nm) suspension was added to 3.0 ml of a 10 mmol/l citrate-disodium hydrogen phosphate buffer (pH: 5.5, 0.8% BSA) containing LVFX-BSA (binding ratio: 3) at 20μg/ml that was prepared by the method described in the section entitled "(1) Preparation of antigen for immunization and antigen for screening" in Example 1(I). The mixture was stirred at 4°C for two hours. After centrifuging the mixture at 4°C and 13,000 rpm for 30 minutes, the supernatant was removed, and suspended in a 5 mmol/l MOPS buffer (pH: 7.0, 0.1% BSA) in an equal volume. After ultrasonic dispersion, the resulting solution was diluted with a 5 mmol/l MOPS buffer (pH: 7.0) so that the absorbance at 280 nm was 1.1 Abs to obtain a second reagent. The second reagent includes "(b) a synthetic polyvalent antigen obtained by immobilizing two or more molecules of an antigen-support composite that includes the compound shown by the formula (I) on a solid phase" recited in (21).
(iii) A levofloxacin solution having a concentration 0.0 μg/ml, 5.0 μg/ml, 10 μg/ml, or 20 μg/ml was prepared in the same manner as in the section entitled "(9) Preparation of compound solution used for competitive ELISA" in Example 1 (I).

(2) Measurement procedure

Each levofloxacin aqueous solution was subjected to the measurement using a 7170S automated analyzer (manufactured by Hitachi, Ltd.). Specifically, 4 μl of the levofloxacin aqueous solution of different concentrations was added to 20 μl each of the first reagent. The mixture was heated at 37°C for 5 minutes. After the addition of 180 μl of the second reagent, a change in absorbance (700 nm) at 19 to 34 photometric points was measured at 37°C.

(II) Results

The levofloxacin aqueous solution was subjected to the measurement, and a change in absorbance corresponding to each levofloxacin concentration was plotted. The plot approximate expression had an excellent linearity within the measurement range. It was thus confirmed that the measurement method according to the present invention can be used to quantitatively determine the compound shown by the formula (I) in a sample (FIG. 15).

Example 10

Synthetic polyvalent antigen in latex agglutination inhibition immunoassay (antibody-immobilized latex)(binding ratio)

This example discusses the ofloxacin binding ratio of "synthetic polyvalent antigen" in "(20) An immunoassay for detecting the compound shown by the formula (I) in a sample, comprising competitively reacting a synthetic polyvalent antigen and the compound shown by the formula (I) in the sample with the antibody according to any one of (1) to (11) that is immobilized on a solid phase".

(I) Materials and methods

(1) Preparation of reagent

A coupling reaction with BSA was performed, using a solution having a concentration of 2.2, 5.4, 8.7, 11.9, 15.2, 18.4, or 21.7 mg/2ml as the levofloxacin solution in the method described in the section entitled "(1) Preparation of antigen for immunization and antigen for screening" in Example 1(I). The LVFX-BSA binding ratio of the resulting coupling product was determined by the absorbance measurement of Example 1(I)(1)(v).

(2) Measurement procedure

Each LVFX-BSA was used as the synthetic polyvalent antigen. A levofloxacin aqueous solution having a concentration of 0.0 μg/ml or 16.0 μg/ml that was prepared in the same manner as in the section entitled "(9) Preparation of compound solution used for competitive ELISA" in Example 1(I) was subjected to the measurement in accordance with the method described in "(I) Latex agglutination inhibition immunoassay (antibody-immobilized latex)" in Example 8. The difference in absorbance between the cases of levofloxacin concentration of 0.0 μg/ml and 16.0 μg/ml was calculated. The levofloxacin concentration and the binding ratio were plotted on a graph.

(II) Results

The binding ratio of the resulting coupling product is indicated by 2.2→3.9, 5.4→5.0, 8.7→6.2, 11.9→8.1, 15.2→10.2, 18.4→13.0, and 21.7→16.7 (levofloxacin solution concentration (mg/ml)→binding ratio). It was thus confirmed that the LVFX-BSA binding ratio increased depending on the amount of LVFX reacted with one molecule of the BSA carrier (FIG. 16).
The difference in absorbance between the cases of levofloxacin concentration of 0.0 μg/ml and 16.0 μg/ml was calculated. The difference in absorbance and the binding ratio were plotted on a graph. The difference in absorbance increased as the LVFX-BSA binding ratio increased. When using latex agglutination inhibition immunoassay, an antigen in a sample could be measured with higher sensitivity as the difference between the absorbance when the antigen was not present in the sample (concentration: 0) and the absorbance when the antigen was present in the sample increased. Good sensitivity was obtained under the conditions employed in this example when the binding ratio was 8 to 15, and excellent sensitivity was obtained when the binding ratio was 15 or more (FIG. 17).

INDUSTRIAL APPLICABILITY

The present invention relates to antibodies to ofloxacin, and methods of producing the same. The present invention also relates to the immunoassay (e.g., radioimmunoassay, enzyme immunoassay, or particle agglutination inhibition immunoassay) using the above antibodies. According to the present invention, the amount of ofloxacin (i.e., strong antibiotic) present in a body can be promptly and accurately measured at a clinical site.

<Reference to deposited biological material>

(1) Antibody number: 77201

(a) Name and address of the depositary institution with which the deposit was made
The International Patent Organism Depositary, National Institute of Advanced Industrial Science and Technology AIST Tsukuba Center 6, 1-1, Higashi 1-chore, Tsukuba-shi, Ibaraki-ken, Japan (postal code: 305-8566)
(b) Date of deposit of the biological material with the institution specified in (a) September 11, 2007 (original

deposit date)
September 5, 2008 (date on which the transferred sample was received by the international depositary authority)
(c) Accession number given to the deposit by the institution specified in (a) FERM BP-11010

(2) Antibody number: 77202

(a) Name and address of the depositary institution with which the deposit was made
The International Patent Organism Depositary, National Institute of Advanced Industrial Science and Technology
AIST Tsukuba Center 6, 1-1, Higashi 1-chome, Tsukuba-shi, Ibaraki-ken, Japan (postal code: 305-8566)
(b) Date of deposit of the biological material with the institution specified in (a) September 11, 2007 (original deposit date)
September 5, 2008 (date on which the transferred sample was received by the international depositary authority)
(c) Accession number given to the deposit by the institution specified in (a) PERM BP-11011

(3) Antibody number: 77204

(a) Name and address of the depositary institution with which the deposit was made
The International Patent Organism Depositary, National Institute of Advanced Industrial Science and Technology
AIST Tsukuba Center 6, 1-1, Higashi 1-come, Tsukuba-shi, Ibaraki-ken, Japan (postal code: 305-8566)
(b) Date of deposit of the biological material with the institution specified in (a) September 11, 2007 (original deposit date)
September 5, 2008 (date on which the transferred sample was received by the international depositary authority)
(c) Accession number given to the deposit by the institution specified in (a) PERM BP-11012

(4) Antibody number: 77205

(a) Name and address of the depositary institution with which the deposit was made
The International Patent Organism Depositary, National Institute of Advanced Industrial Science and Technology
AIST Tsukuba Center 6, 1-1, Higashi 1-chome, Tsukuba-shi, Ibaraki-ken, Japan (postal code: 305-8566)
(b) Date of deposit of the biological material with the institution specified in (a) September 11, 2007 (original deposit date)
September 5, 2008 (date on which the transferred sample was received by the international depositary authority)
(c) Accession number given to the deposit by the institution specified in (a) FERM BP-11013

(5) Antibody number: 77206

(a) Name and address of the depositary institution with which the deposit was made
The International Patent Organism Depositary, National Institute of Advanced Industrial Science and Technology
AIST Tsukuba Center 6, 1-1, Higashi 1-chome, Tsukuba-shi, Ibaraki-ken, Japan (postal code: 305-8566)
(b) Date of deposit of the biological material with the institution specified in (a) September 11, 2007 (original deposit date)
September 5, 2008 (date on which the transferred sample was received by the international depositary authority)
(c) Accession number given to the deposit by the institution specified in (a) FERM BP-11014

(6) Antibody number: 77207

(a) Name and address of the depositary institution with which the deposit was made
The International Patent Organism Depositary, National Institute of Advanced Industrial Science and Technology
AIST Tsukuba Center 6, 1-1, Higashi 1-chome, Tsukuba-shi, Ibaraki-ken, Japan (postal code: 305-8566)
(b) Date of deposit of the biological material with the institution specified in (a) September 11, 2007 (original deposit date)
September 5, 2008 (date on which the transferred sample was received by the international depositary authority)
(c) Accession number given to the deposit by the institution specified in (a) FERM BP-11015

(7) Antibody number: 77209

(a) Name and address of the depositary institution with which the deposit was made
The International Patent Organism Depositary, National Institute of Advanced Industrial Science and Technology

AIST Tsukuba Center 6, 1-1, Higashi 1-chome, Tsukuba-shi, Ibaraki-ken, Japan (postal code: 305-8566)
(b) Date of deposit of the biological material with the institution specified in (a) September 11, 2007 (original deposit date)
September 5, 2008 (date on which the transferred sample was received by the international depositary authority)
(c) Accession number given to the deposit by the institution specified in (a) FERM BP-11016

BRIEF DESCRIPTION OF THE DRAWING

FIG. 1 shows the measurement results determined by antigen-immobilized ELISA of the antibody titre in the mouse antiserum.
FIG. 2 shows the measurement results, determined by competitive ELISA, of the relationship between the amount of free levofloxacin and the reactivity (Abs.), with the immobilized antigen, of the antibody in the mouse antiserum.
FIG. 3 shows the measurement results, determined by competitive ELISA, of the relationship between the amount of free levofloxacin and the reactivity (cross-reactivity rate) of each monoclonal antibody with the immobilized antigen.
FIG. 4 shows a calibration curve determined by competitive ELISA using the standard solution.
FIG. 5 shows the results of the dilution linearity test at each serum concentration.
FIG. 6 shows a correlation between the measured value and the theoretical value determined by competitive ELISA using samples having known concentrations.
FIG. 7 shows the effects of addition of saliva to the competitive ELISA measurement system.
FIG. 8 shows the effects of addition of plasma to the competitive ELISA measurement system.
FIG. 9 shows the effects of addition of plasma to the competitive ELISA measurement system using a conventional antibody.
FIG. 10 shows calibration curves determined by the one-step competitive ELISA using the standard solution.
FIG. 11 shows a calibration curve determined by latex agglutination inhibition immunoassay (antibody-immobilized latex) using the standard solution.
FIG. 12 shows a correlation between the measured value and the theoretical value determined by latex agglutination inhibition immunoassay (antibody-immobilized latex) that uses levofloxacin-containing human serum having known concentrations.
FIG. 13 shows a correlation between the measured value and the theoretical value determined by latex agglutination inhibition immunoassay (antibody-immobilized latex) that uses levofloxacin-containing human plasma having known concentrations.
FIG. 14 shows a correlation between the levofloxacin concentration in levofloxacin-containing saliva having a known concentration and a change in absorbance determined by latex agglutination inhibition immunoassay (antibody-immobilized latex).
FIG. 15 shows a calibration curve determined by latex agglutination inhibition immunoassay (antigen-immobilized latex) using the standard solution.
FIG. 16 shows a correlation between the amount of levofloxacin used as the raw material and the BSA binding ratio when preparing a synthetic polyvalent antigen using BSA as a carrier.
FIG. 17 shows a correlation between the levofloxacin-BSA binding ratio and the difference between the absorbances when the levofloxacin concentration in the sample is 0.0 (μg/ml and 16 μg/ml, determined by latex agglutination inhibition immunoassay (antibody-immobilized latex).

**Claims**

1. An antibody that reacts with a compound shown by the following formula (I),

[Chem 1]

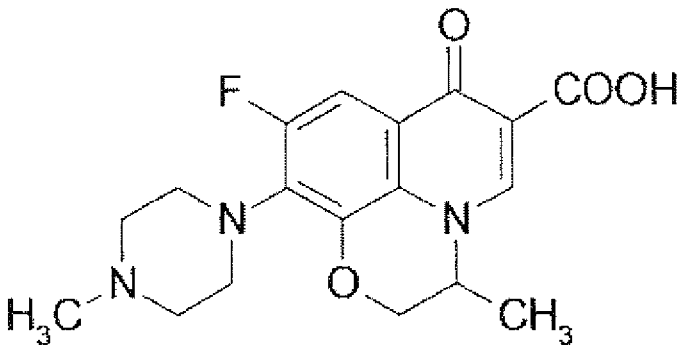

(I)

but does not cross-react with an N-oxide metabolite and/or a desmethyl metabolite thereof.

2. The antibody according to claim 1, wherein the antibody strongly reacts with an S-isomer of the compound shown by the formula (I).

3. The antibody according to claim 1, wherein the antibody strongly reacts with an R-isomer of the compound shown by the formula (I).

4. The antibody according to claim 1, wherein the antibody strongly reacts with a racemic body of the compound shown by the formula (I).

5. The antibody according to claim 1, wherein the antibody does not cross-react with one or more compounds selected from the group consisting of diclofenac sodium, nabumetone, flurbiprofen, ketoprofen, loxoprofen sodium, oxaprozin, naproxen, ibuprofen, carbocisteine, salicylamide, acetaminophen, anhydrous caffeine, methylenedisalicylic acid, promethazine, and theophylline.

6. The antibody according to claim 1, wherein the antibody does not cross-react with, or weakly reacting with, new quinolone antibiotics other than the compound shown by the formula (I).

7. The antibody according to any one of claims 1 to 6, wherein the reactivity of the antibody with the compound shown by the formula (I) does not change due to the presence of a biological sample-derived component.

8. The antibody according to any one of claims 1 to 7, wherein the biological sample-derived component is a serum component, a plasma component, or a salivary component.

9. The antibody according to any one of claims 1 to 6, wherein the reactivity of the antibody with the compound shown by the formula (I) does not change due to binding between the compound shown by the formula (I) and a serum component.

10. The antibody according to any one of claims 1 to 9, wherein the antibody is a monoclonal antibody.

11. The monoclonal antibody according to claim 10, wherein the monoclonal antibody is specific to the compound shown by the formula (I), and satisfying a reaction condition whereby, in a reaction system in which the compound shown by the formula (I) in a sample and an N-oxide metabolite and/or a desmethyl metabolite of the compound shown by the formula (I) in a sample are present so that an immune reaction between the compound shown by the formula (I) immobilized on a solid phase and the antibody is inhibited, the 50% inhibitory concentration of the compound shown by the formula (I) in the sample is higher than the 50% inhibitory concentration of the N-oxide metabolite and/or the desmethyl metabolite of the compound shown by the formula (I) in the sample.

12. A hybridoma that produces the monoclonal antibody according to claim 10 or 11.

**13.** An antigen for producing the antibody according to claim 1, wherein the antigen is produced by binding a carrier protein to the compound shown by the formula (I) through the 6-position carboxyl group.

**14.** The antigen according to claim 13, wherein the carrier protein is BSA or transferrin.

**15.** The antigen according to claim 13 or 14, wherein 12 to 14 molecules of the compound shown by the formula (I) are bound to one molecule of the carrier protein.

**16.** An immunization method comprising immunizing an animal with the antigen according to any one of claims 13 to 15.

**17.** An antibody screening method comprising: reacting an antibody obtained by immunizing an animal with the antigen according to any one of claims 13 to 15 with the compound shown by the formula (I) that is immobilized on a solid phase in the presence of a compound for which it is desired to determine cross-reactivity, and; comparing the resulting reactivity with the reactivity in the absence of the compound for which it is desired to determine cross-reactivity to select a desired antibody.

**18.** An antibody screening method comprising: reacting an antibody obtained by immunizing an animal with the antigen according to any one of claims 13 to 15 with the compound shown by the formula (I) that is immobilized on a solid phase in the presence of a biological sample-derived component, and; comparing the resulting reactivity with the reactivity in the absence of the biological sample-derived component to select a desired antibody.

**19.** An immunoassay for detecting the compound shown by the formula (I) in a sample, comprising using the antibody according to any one of claims 1 to 11.

**20.** An immunoassay for detecting the compound shown by the formula (I) in a sample, comprising competitively reacting a synthetic polyvalent antigen and the compound shown by the formula (I) in the sample with the antibody according to any one of claims 1 to 11 that is immobilized on a solid phase.

**21.** An immunoassay for detecting the compound shown by the formula (I) in a sample, comprising competitively reacting an immobilized synthetic polyvalent antigen and the compound shown by the formula (I) in the sample with the antibody according to any one of claims 1 to 11.

**22.** An immunoassay for detecting the compound shown by the formula (I) in a sample, comprising competitively reacting an immobilized synthetic polyvalent antigen and the compound shown by the formula (I) in the sample with the antibody according to any one of claims 1 to 11 that is immobilized on a solid phase.

**23.** The immunoassay according to any one of claims 19 to 22, wherein the solid phase is a latex particle.

**24.** The immunoassay according to any one of claims 19 to 23, wherein the competitive reaction is measured by an agglutination inhibition method.

**25.** The immunoassay according to any one of claims 19 to 24, wherein the sample is blood, serum, plasma, urine, saliva, sputum, lacrimal fluid, otorrhea, or prostatic fluid.

**26.** The immunoassay according to claim 25, wherein the sample is a sample collected from a patient who has been administered the compound shown by the formula (I).

**27.** A reagent for immunoassay for detecting the compound shown by the formula (I) in a sample, the reagent comprising:

(a) the antibody according to any one of claims 1 to 11, or the antibody according to any one of claims 1 to 11 that is immobilized on a solid phase; and
(b) a synthetic polyvalent antigen or an immobilized synthetic polyvalent antigen.

[Figure 1]

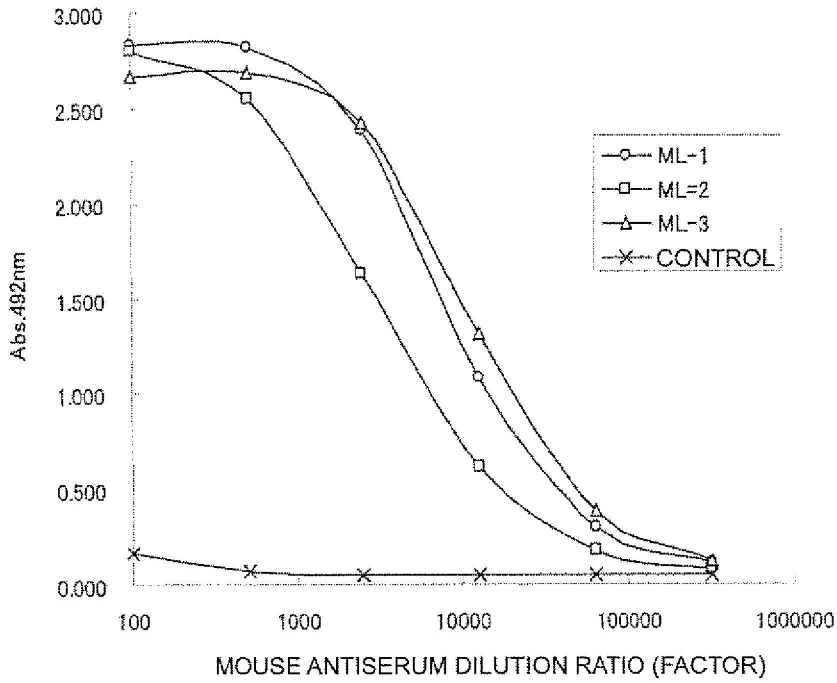


[Figure 2]

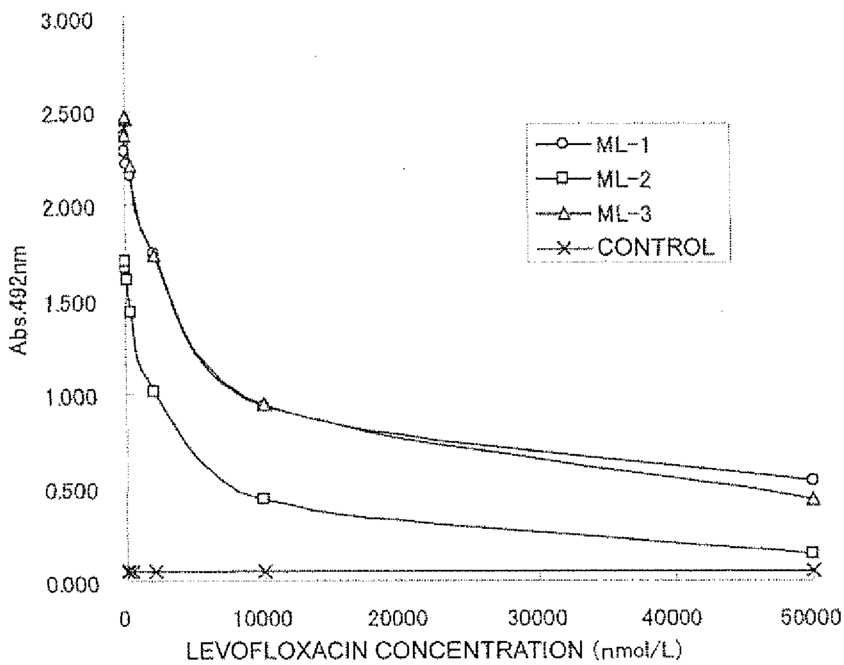

[Figure 3]

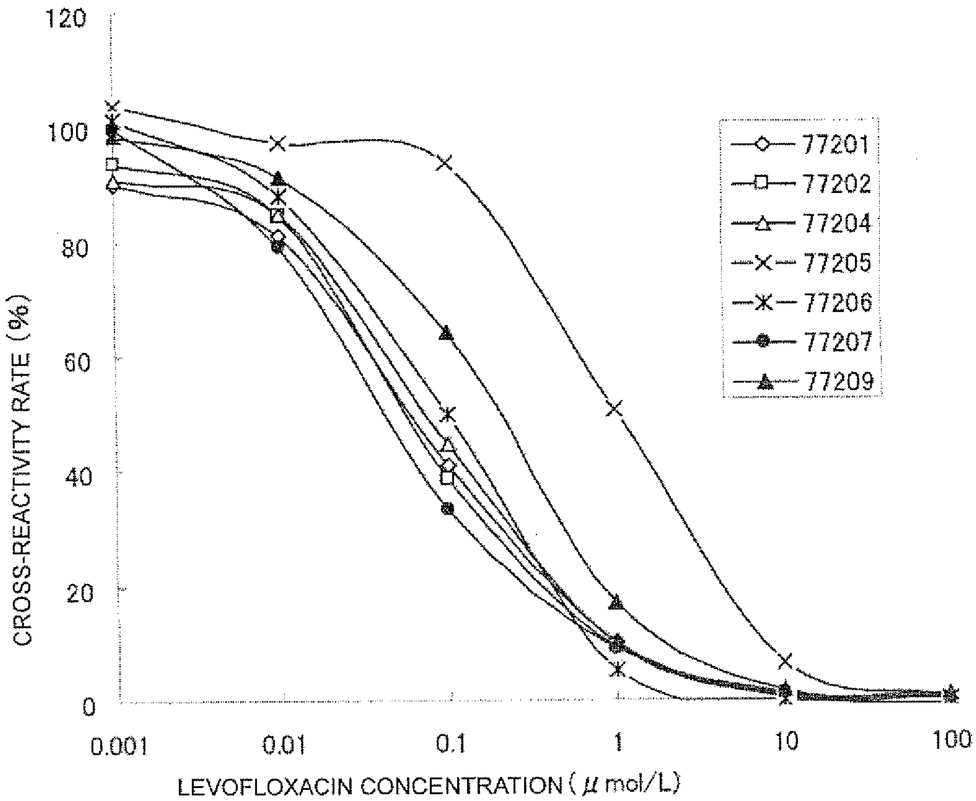


[Figure 4]

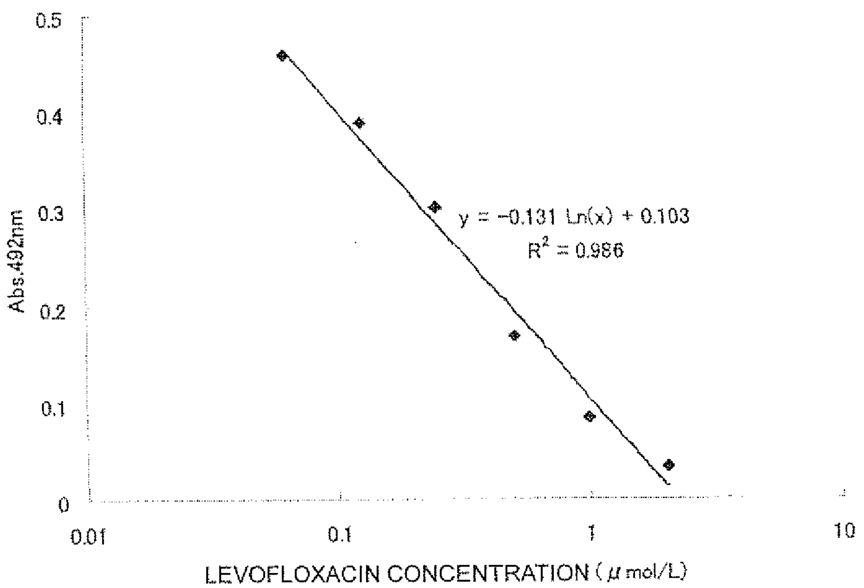

[Figure 5]

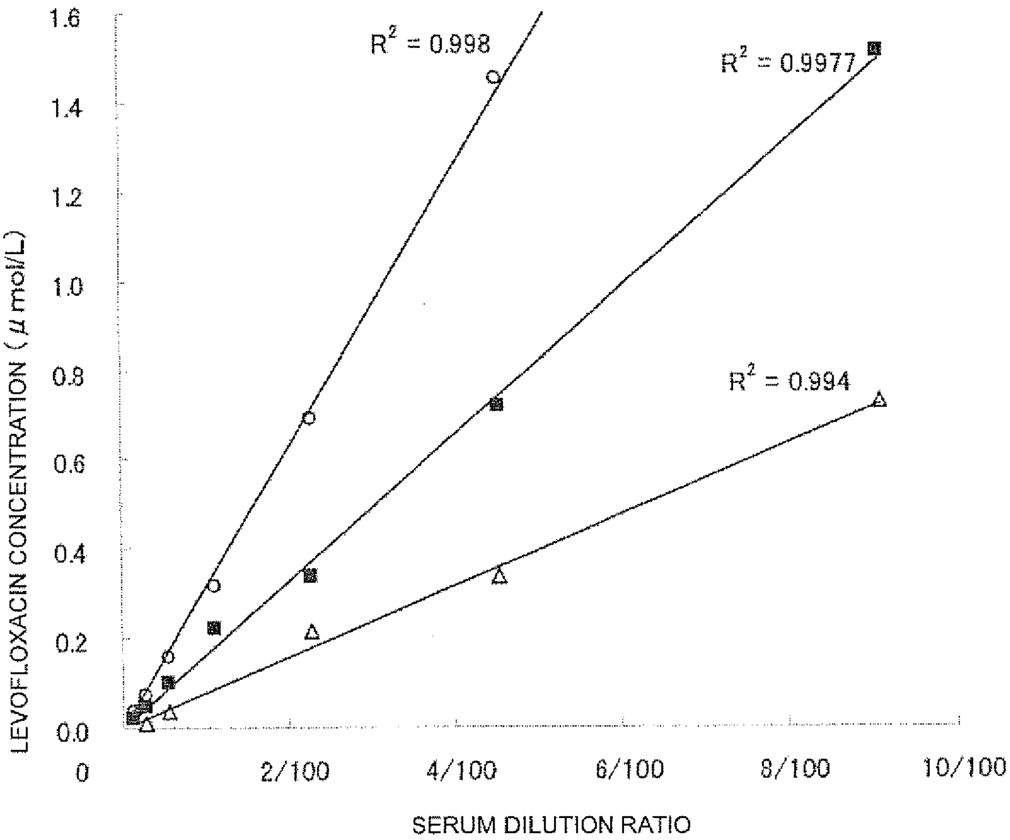
R2 = 0.998
R2 = 0.9977
R2 = 0.994
1.6
1.4
1.2
1.0
0.8
0.6
0.4
0.2
0.0
LEVOFLOXACIN CONCENTRATION (μmol/L)
0
2/100
4/100
6/100
8/100
10/100
SERUM DILUTION RATIO

[Figure 6]

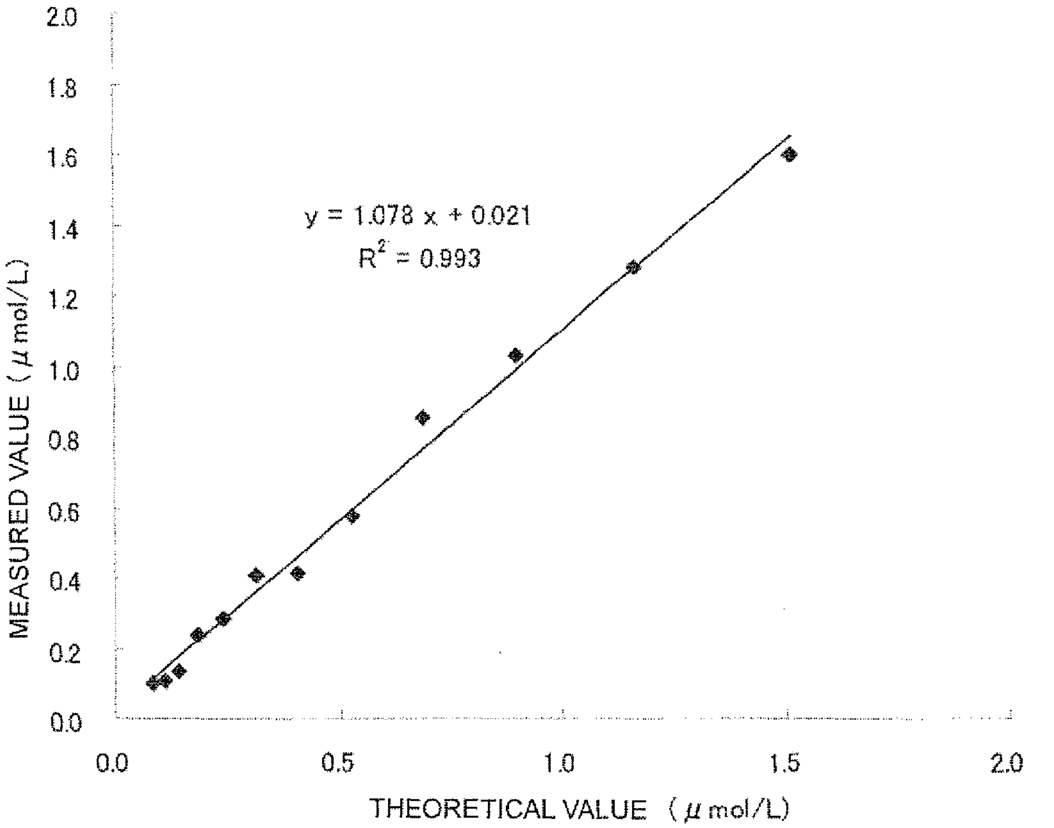
y = 1.078 x + 0.021
R² = 0.993
MEASURED VALUE (μmol/L)
THEORETICAL VALUE (μmol/L)
2.0
1.8
1.6
1.4
1.2
1.0
0.8
0.6
0.4
0.2
0.0
0.0
0.5
1.0
1.5
2.0

[Figure 7]

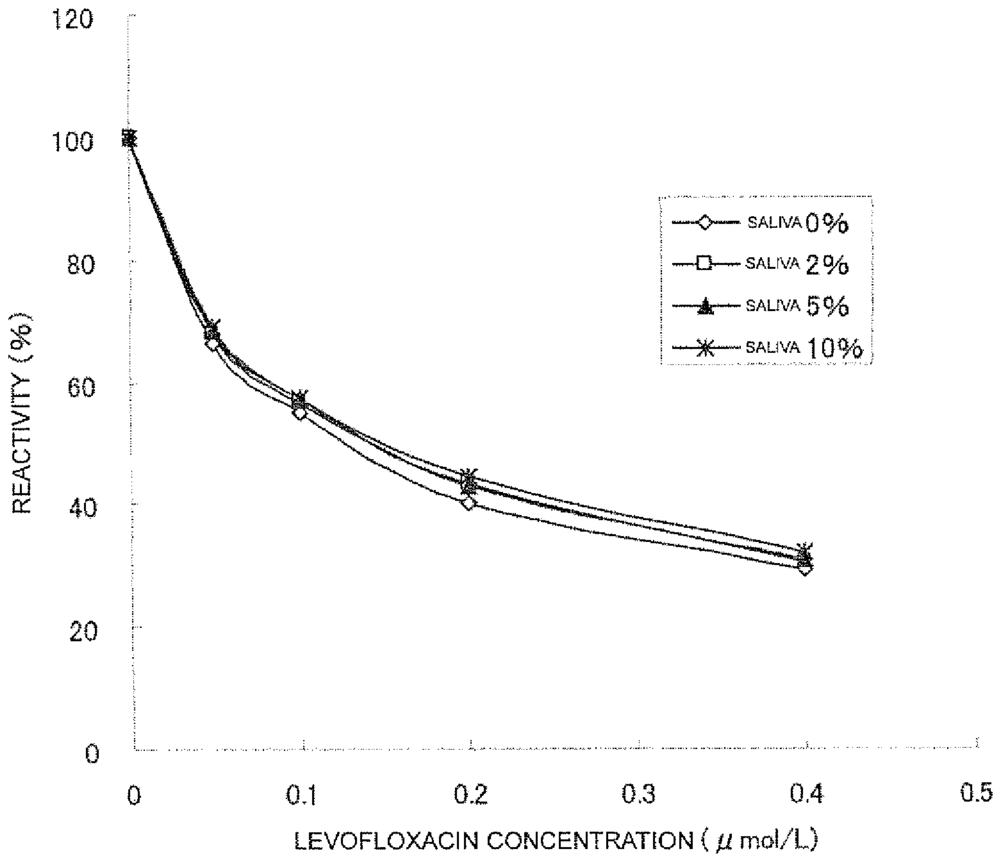
120
100
80
60
40
20
0
REACTIVITY (%)
0
0.1
0.2
0.3
0.4
0.5
LEVOFLOXACIN CONCENTRATION ( μ mol/L)
SALIVA 0%
SALIVA 2%
SALIVA 5%
SALIVA 10%

[Figure 8]

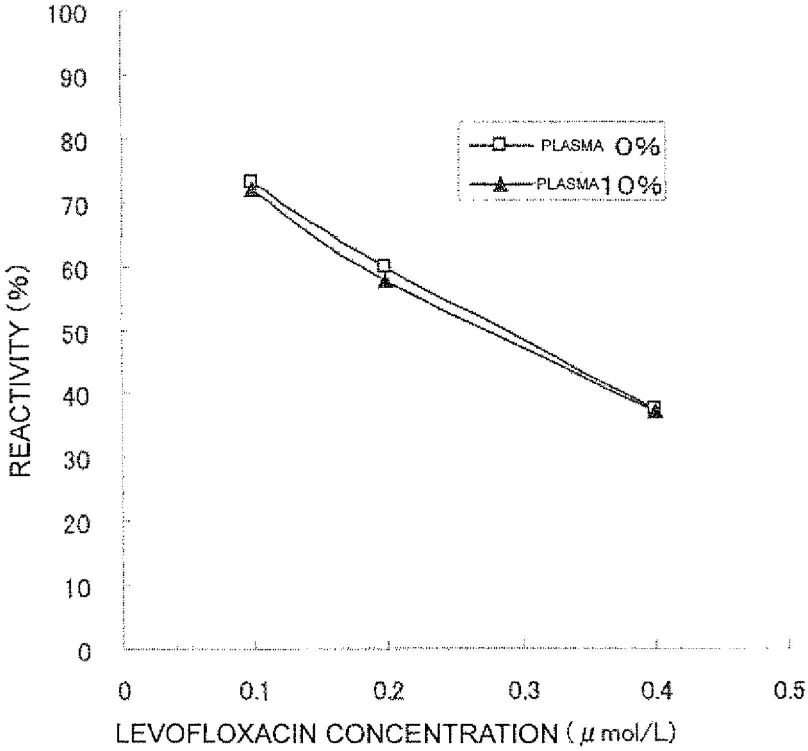


[Figure 9]

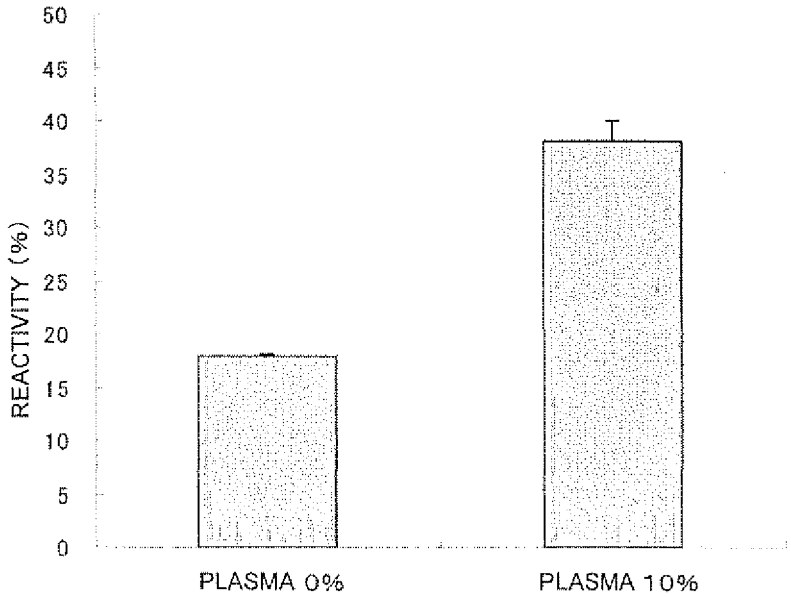

[Figure 10]

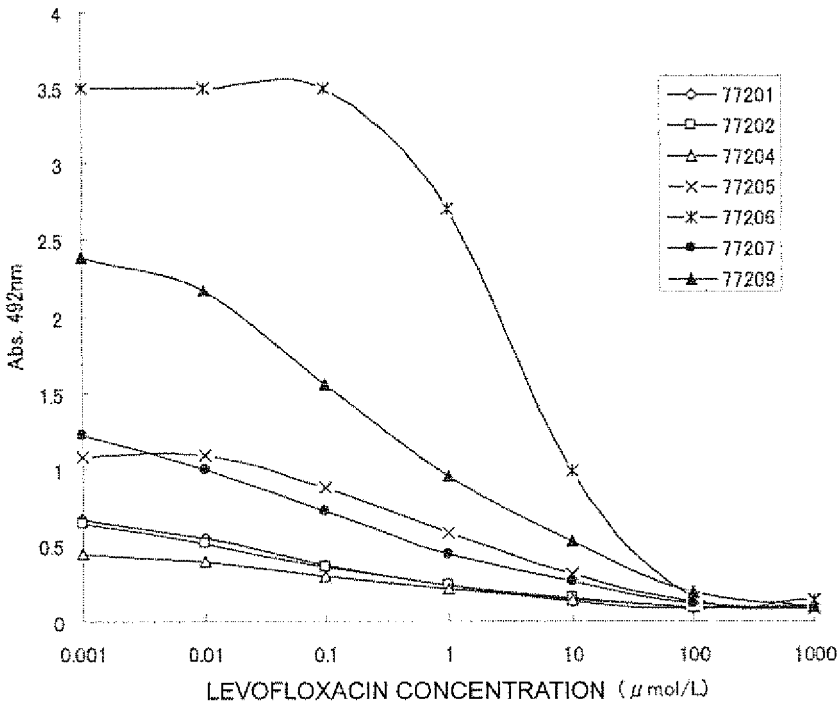


[Figure 11]

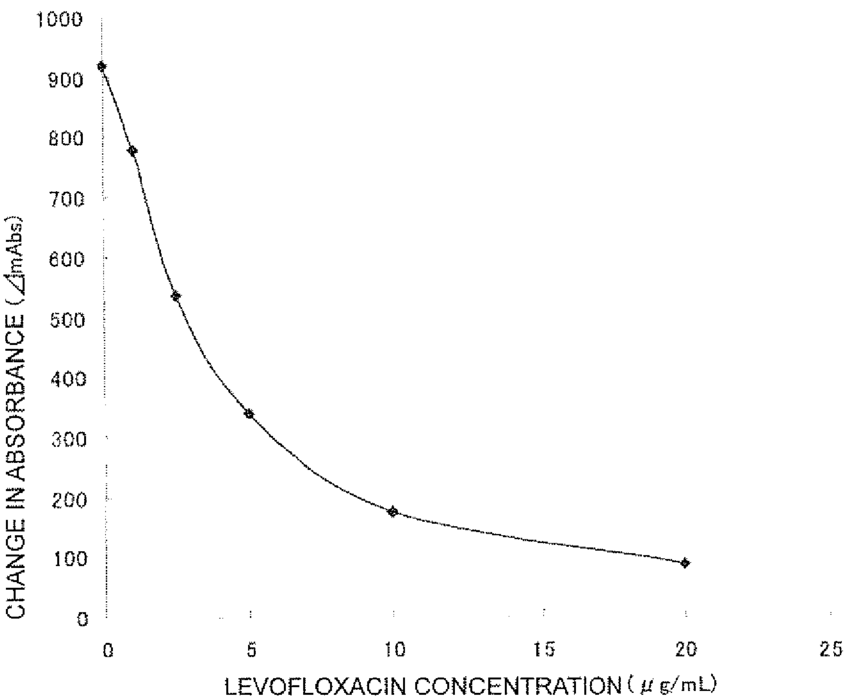

[Figure 12]

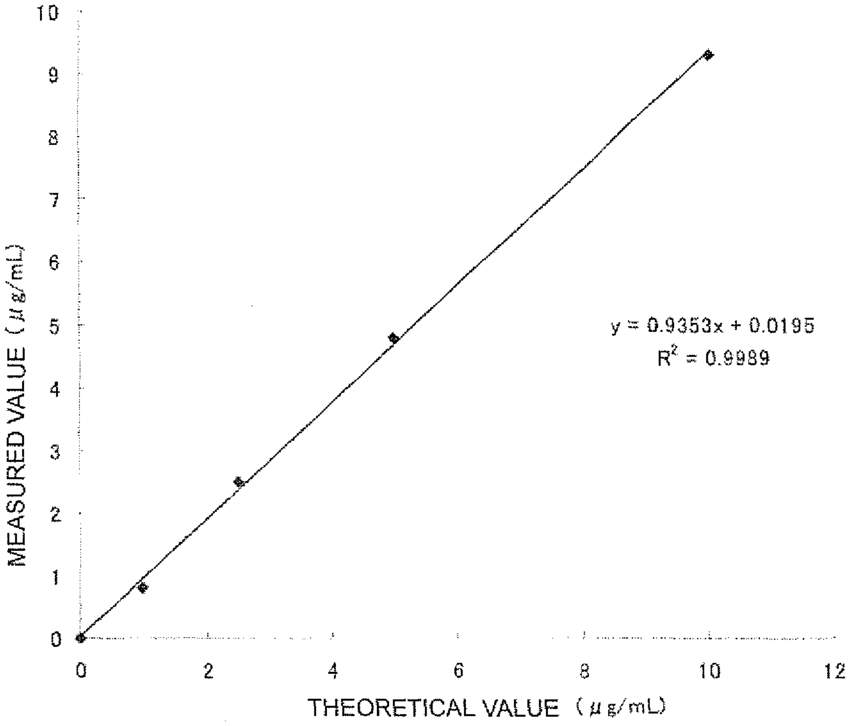


[Figure 13]

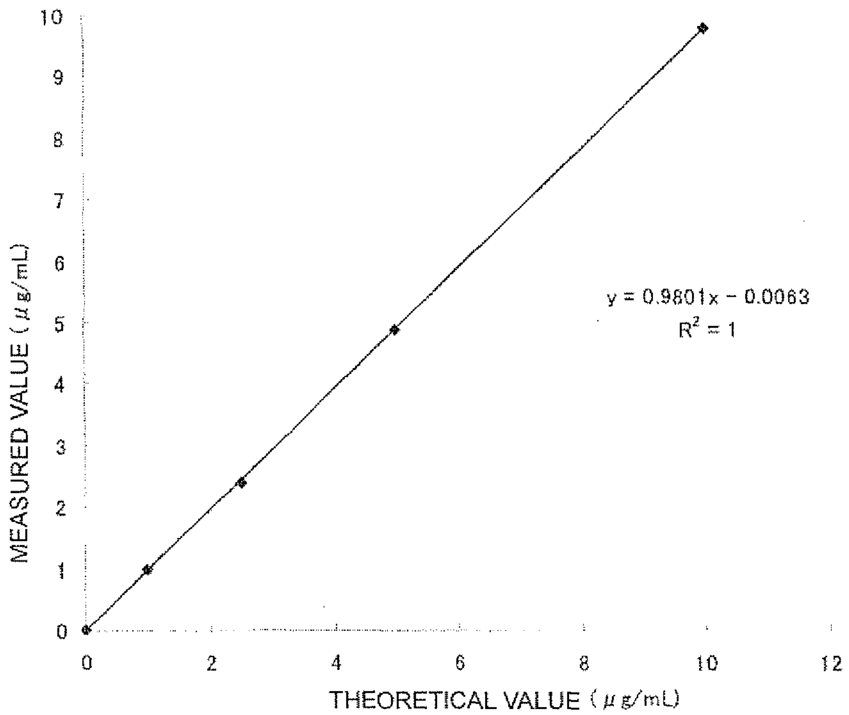

[Figure 14]

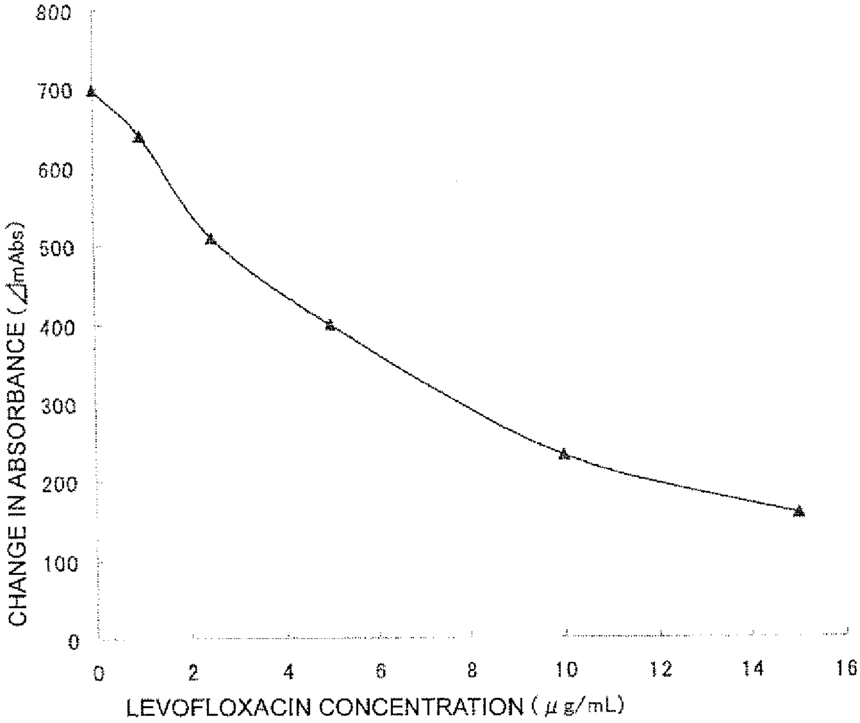


[Figure 15]

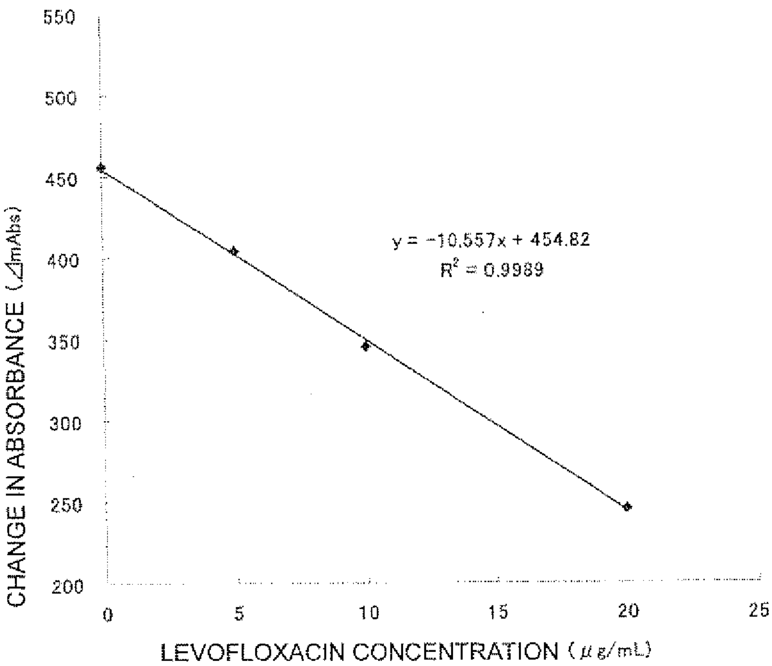

[Figure 16]

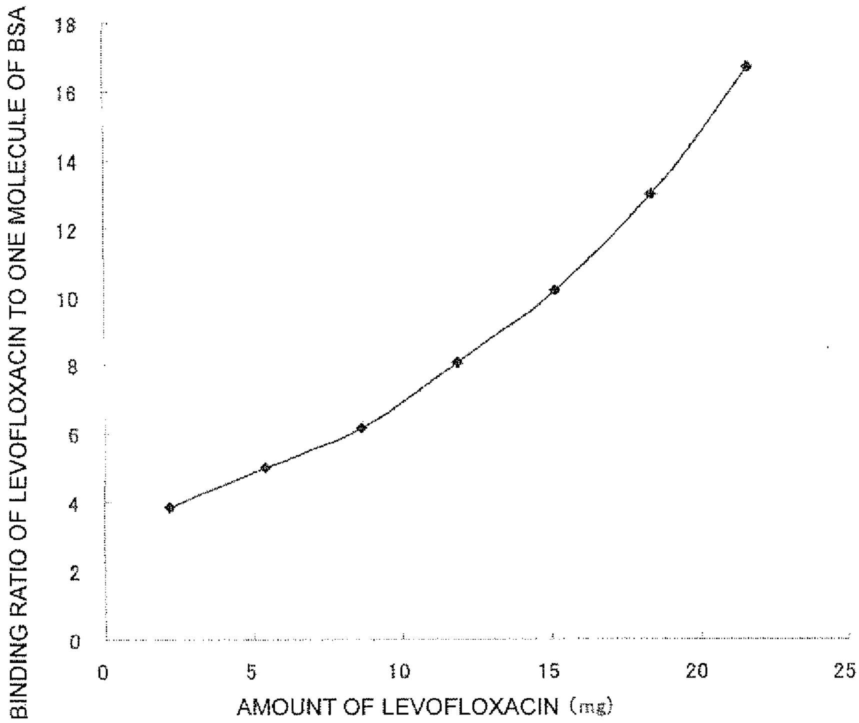


[Figure 17]

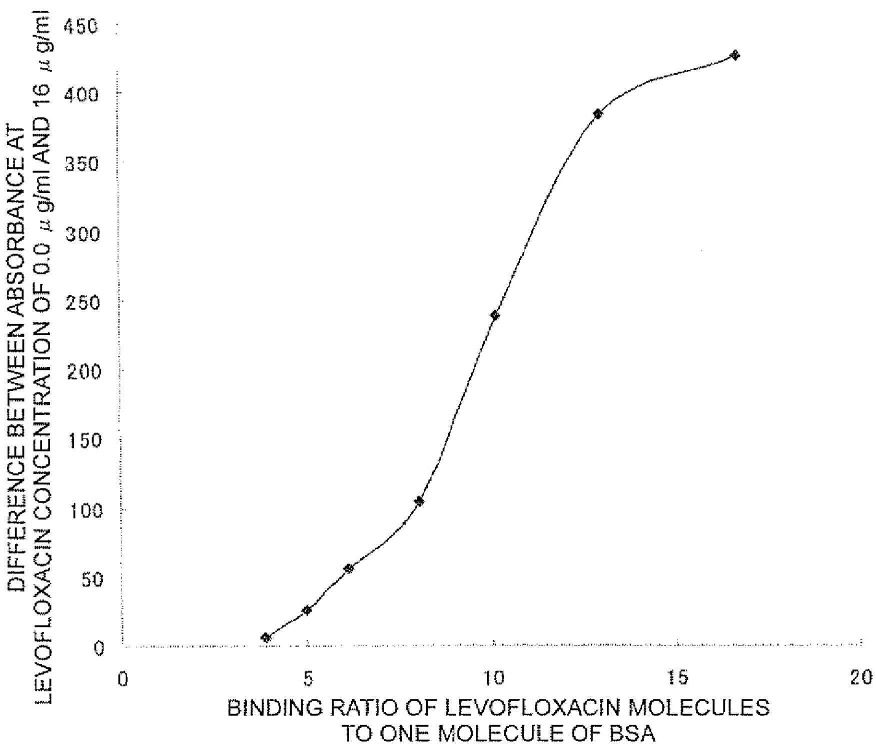

**INTERNATIONAL SEARCH REPORT**

International application No.
PCT/JP2008/072783

A. CLASSIFICATION OF SUBJECT MATTER
*C07K16/44*(2006.01)i, *C07K14/76*(2006.01)i, *C07K14/79*(2006.01)i, *C12N5/10* (2006.01)i, *C12N15/02*(2006.01)i, *G01N33/50*(2006.01)i, *G01N33/53*(2006.01)i, *G01N33/577*(2006.01)i, *C12P21/08*(2006.01)n

According to International Patent Classification (IPC) or to both national classification and IPC

B. FIELDS SEARCHED

Minimum documentation searched (classification system followed by classification symbols)
C07K16/44, C07K14/76, C07K14/79, C12N5/10, C12N15/02, G01N33/50, G01N33/53, G01N33/577, C12P21/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus(STN), REGISTRY(STN), MEDLINE(STN), JSTPlus(JDreamII)

C. DOCUMENTS CONSIDERED TO BE RELEVANT

| Category* | Citation of document, with indication, where appropriate, of the relevant passages | Relevant to claim No. |
|---|---|---|
| X/Y | Wang Z, et al., Development of a monoclonal antibody-based broad-specificity ELISA for fluoroquinolone antibiotics in foods and molecular modeling studies of cross-reactive compounds. Anal Chem., 2007 Jun, Vol.79, No.12, p.4471-4483. (Epub 2007 May) | 13,14/1-12, 15,17-27 |
| Y | JP 7-267999 A (Daiichi Pharmaceutical Co., Ltd.), 17 October, 1995 (17.10.95), (Family: none) | 1-12,15, 17-27 |
| A | JP 2007-063180 A (Frontier Institute Co., Ltd.), 15 March, 2007 (15.03.07), (Family: none) | 1-27 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 January, 2009 (06.01.09) | 20 January, 2009 (20.01.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2008/072783

C (Continuation). DOCUMENTS CONSIDERED TO BE RELEVANT

| Category* | Citation of document, with indication, where appropriate, of the relevant passages | Relevant to claim No. |
|---|---|---|
| A | Jun'ichi MITSUYAMA, "Quinolone-kei Kokin 'yaku no Kiso", Folia Pharmacol.Jpn., 2007 Oct, Vol.130, No.4, pages 287 to 293 | 1-27 |

Form PCT/ISA/210 (continuation of second sheet) (April 2007)

**INTERNATIONAL SEARCH REPORT**

International application No.
PCT/JP2008/072783

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 16
because they relate to subject matter not required to be searched by this Authority, namely:
The invention as set forth in the above claim pertains to methods for treatment of the human body by diagnostic methods.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
When considering the description of this specification, an antibody included in claims 1-12 and 19-27 is considered to be produced based on an antigen described in claims 13-15, 17 and 18, however, the antibody produced based on the antigen has already been known (for example, Anal Chem., 2007, Jun., Vol. 79, No. 12, pp. 4471-4483, etc.). Therefore, these inventions are not considered to be a group of inventions so linked as to form a single general inventive concept, and this application is considered to include two inventions.

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007063180 A
- JP 7267999 A


### Non-patent literature cited in the description


- Antibodies, A Laboratory Manual. Cold Spring Harbor Laboratory Press, 1988
- immunoassay for clinical examination - technology and application. **Rinsho Byori.** Japanese Society of Laboratory Medicine. 1983
- Enzyme Immunoassay. Igaku-Shoin, Ltd, 1987
- Enzyme Immunoassay. Protein, Nucleic Acid and Enzyme. Kyoritsu Shuppan, Co., Ltd, 1987